# FASCICULE DE BREVET EUROPEEN

(11) **EP 1 651 677 B1**
(45) Date de publication et mention de la délivrance du brevet: **10.11.2010**
(21) Numéro de dépôt: 04785991.3
(22) Date de dépôt: 22.07.2004
(51) Int. Cl.: C08B 37/10, A61K 31/727

(54) **MELANGES D'OLIGOSACCHARIDES DERIVES D'HEPARINE, LEUR PREPARATION ET LES COMPOSITIONS PHARMACEUTIQUES LES CONTENANT**
MISCHUNGEN AUS VON HEPARIN ABGELEITETEN OLIGOSACCHARIDEN, HERSTELLUNG DAVON UND PHARMAZEUTISCHE ZUSAMMENSETZUNGEN, DIE DIE MISCHUNGEN ENTHALTEN
HEPARIN-DERIVED OLIGOSACCHARIDE MIXTURES, PREPARATION THEREOF AND PHARMACEUTICAL COMPOSITIONS CONTAINING SAID MIXTURES

(30) Priorité: 24.07.2003 FR 0309041
(43) Date de publication de la demande: 03.05.2006
(73) Titulaire: Aventis Pharma S.A., 92160 Antony (FR)
(72) Inventeur: LAUX, Volker, 55128 Mainz (DE); MOURIER, Pierre, F-94220 Charenton le Pont (FR); VISKOV, Christian, F-91130 Ris Orangis (FR)
(74) Mandataire: Jacobson, Claude
(86) Numéro de dépôt international: PCT/FR2004/001943
(87) Numéro de publication internationale: WO 2005/010051

(56) Documents cités:
- EP-A- 0 027 089
- WO-A-01/72762
- WO-A-02/08295
- WO-A-2004/033503
- US-B1- 6 384 021

## Description

La présente invention concerne des mélanges d'oligosaccharides dérivés d'héparine ayant un poids moléculaire moyen de 1800 à 2400 Daltons, caractérisés par une forte activité anti-Xa (aXa) et une absence d'activité anti-IIa (aIIa), leur procédé de préparation et les compositions pharmaceutiques les contenant.

L'héparine est un mélange de mucopolysaccharides sulfatés d'origine animale, utilisée notamment pour ses propriétés anticoagulantes et antithrombotiques.

L'héparine présente cependant des inconvénients qui limitent les conditions de son utilisation. En particulier, son activité anticoagulante importante (aIIa) peut occasionner des hémorragies. (Seminars in Thrombosis and Hemostasis, vol 5 sup. 3 (1999)).

Des héparines de bas poids moléculaire, notamment obtenues par dépolymérisation basique d'esters d'héparine et actuellement commercialisées telle que l'Enoxaparine présentent également une activité aIIa importante

Plus récemment, des héparines de très bas poids moléculaires ont été décrites dans l'art antérieur. Par example, dans le brevet US 6,384,021 les produits présentent une activité anti-Xa comprise entre 100 et 120 UI/mg, une activité anti-IIa comprise entre 2 et 8 UI/mg. Dans les demandes internationales WO02/08295 et WO2004/033503 les produits présentent des activités anti-Xa comprises notamment entre 100 et 190 UI/mg pour des activités anti-IIa inferieures à 5 UI/mg. Cependant, aucune de ces héparines de très bas poids moléculaire ne présentent effectivement une activité anti-Xa supérieur à 190 UI/mg tout en présentant une activité anti-IIa nulle ou pratiquement nulle. (UI = Unité Internationale)

Par activité anti-IIa pratiquement nulle (ou autrement dit, ne présentant pratiquement pas d'activité anti-IIa) on entend, une activité inférieure à 0,2 UI/mg.

L'invention a pour objet des mélanges d'oligosaccharides possédant une activité très sélective vis-à-vis du facteur X activé (facteur Xa) tout en ne présentant pas, ou pratiquement pas, d'activité anti-IIa.

La présente invention a donc pour objet des mélanges d'oligosaccharides possédant la structure générale des polysaccharides constitutifs de l'héparine et présentant les caractéristiques suivantes :
- ils ont un poids moléculaire moyen de 1800 à 2400 Daltons, une activité anti-Xa compris entre 190 UI/mg et 450 UI/mg et une activité anti-IIa inférieure à 0,2 UI/mg.
- les oligosaccharides constitutifs des mélanges
   - contiennent de 2 à 16 motifs saccharidiques,
   - ont un motif acide uronique-4,5 insaturé 2-O-sulfate à l'une de leurs extrémités,
   - et contiennent l'hexasaccharide de formule suivante : 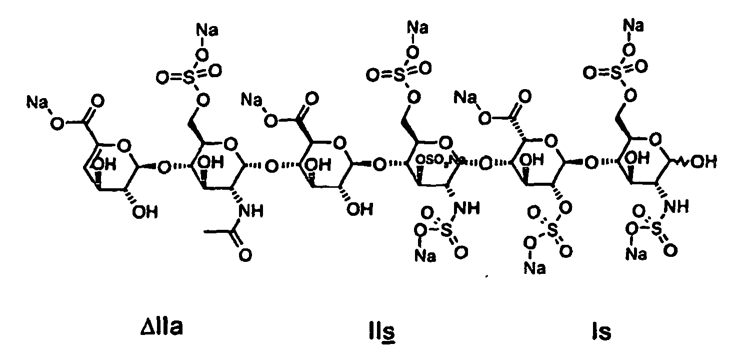 sous forme d'un sel de métal alcalin ou alcalinoterreux.

L'hexasaccharide ΔIIa-IIs-Is contenu dans le mélange d'oligosaccharides décrit dans la présente invention est une séquence fortement affine à l'ATIII et est caractérisé par une activité aXa supérieure à 740 UI/mg.

Comme sel de métal alcalin ou alcalinoterreux, sont préférés les sels de sodium, potassium, calcium et magnésium.

Le poids moléculaire moyen est déterminé par chromatographie liquide haute pression en utilisant deux colonnes en série par exemple celles commercialisées sous le nom de TSK G3000 XL et TSK G2000 XL. La détection est réalisée par réfractométrie. L'éluant utilisé est du nitrate de lithium et le débit est de 0,6 ml/min. Le système est calibré avec des standards préparés par fractionnement de l'Enoxaparine par chromatographie sur gel d'agarose-polyacrylamide (IBF). Cette préparation est réalisée selon la technique décrite par Barrowcliffe et al, Thromb. Res., 12, 27-36 (1977-78) ou D.A. Lane et al , Thromb. Res., 12, 257-271 (1977-78). Les résultats sont calculés à l'aide du logiciel GPC6 (Perkin Elmer). L'activité anti-Xa est mesurée par la méthode amydolytique sur un substrat chromogénique selon le principe décrit par Teien et al, Thromb. Res., 10, 399-410 (1977). Les dosages sont réalisés selon la méthode décrite dans la monographie des héparines de basse masse moléculaire de la pharmacopée européenne en vigueur, à l'exception du tampon de reconstitution : l'albumine dans le tampon Tris-NaCl pH 7.4 est remplacée par du Polyethylene Glycol 6000 (PEG 6000).

La mesure de l'activité anti-Xa est réalisée par rapport à une héparine de très bas poids moléculaire (HTBPM) standard qui titre de 140 à 180 U/mg (sur sec). L'activité de l'HTBPM standard est mesurée par rapport à l'étalon international standard d'héparine de bas poids moléculaire.

Cette HTBPM standard a été préparée selon l'enseignement des demandes de brevet WO WO02/08295 et notamment de WO2004/033503. L'activité de l'HTBPM standard est mesurée par rapport à l'étalon international standard d'héparine de bas poids moléculaire.

L'activité anti-IIa est mesurée par la méthode amidolytique sur un substrat chromogénique selon la méthode décrite dans la monographie des héparines de basse masse moléculaire de la pharmacopée européenne en vigueur. La mesure des activités aIIa est réalisée par rapport à une héparine de trés bas poids moléculaire (HTBPM) standard d'activité mesurée à 2,1 UI/mg. L'activité de l'HTBPM standard est mesurée par rapport à l'étalon international standard d'héparine de bas poids moléculaire.

Selon un mode préféré, le mélange d'oligosaccharides selon l'invention renferme de 20 à 100 % d'une fraction hexasaccharide. En particulier ce mélange contient de 30 à 60 % de fraction hexsaccharide.

Par ailleurs, les mélanges selon l'invention contiennent de 20 à 70 % de l'hexasaccharide ΔIIa-IIs-Is dans la fraction hexasaccharide du mélange d'oligosaccharides. En particulier, cette fraction ΔIIa-ns-Is est présente dans la fraction hexasaccharide à hauteur de 25 à 50 %.

Le pourcentage de la fraction hexasaccharide peut être déterminée de façon analytique par chromatographie liquide haute pression sur colonnes TSK G3000 XL et TSK G2000 XL ou bien par séparation préparative de la fraction hexasaccharide.

Le mélange est dans ce cas chromatographié sur des colonnes remplies de gel de type polyacrylamide agarose. Le mélange est élué par une solution d'hydrogénocarbonate de sodium. De préférence, la solution d'hydrogénocarbonate de sodium est une solution de 0.1 mol/ 1 à 1 mol/l. Encore plus préférentiellement, la séparation est réalisée a une concentration de 1 mole/l. La détection est réalisée par spectrométrie UV (254nm). Après fractionnement, la fraction hexasaccharide en solution dans l'hydrogénocarbonate de sodium est neutralisée par de l'acide acétique glacial. La solution est ensuite concentrée sous pression réduite de façon à obtenir une concentration en acétate de sodium supérieure à 30 % en poids. La fraction d'hexasaccharide est précipitée par addition de 3 à 5 volumes de méthanol. La fraction d'hexasaccharides est récupérée par filtration sur verre fritté n°3. Le mélange d'hexasaccharides obtenu peut être analysé par Chromatographie Liquide Haute Performance (CLHP) pour en déterminer la teneur en hexasaccharide ΔIIa-IIs-Is. L'hexasaccharide ΔIIa-IIs-Is peut être isolé par chromatographie CLHP préparative ou par chromatographie d'affinité sur colonne antithrombine III sepharose selon les techniques utilisées par l'homme du métier (M. Hook, I. Bjork, J. Hopwood and U. Lindahl, F.E B.S letters, vol 656(1) (1976)).

De préférence, les mélanges selon l'invention ont un poids moléculaire moyen compris entre 1900 et 2200 Daltons et en particulier de 1950 à 2150 Daltons.

Selon un mode préféré, le mélange d'oligosaccharides selon l'invention est **caractérisé en ce qu**'il présente une activité anti-Xa comprise entre 190 UI/mg et 410 UI/mg et une activité anti-IIa nulle ou pratiquement nulle. Tout particulièrement l'activité anti-Xa est comprise entre 200 et 300 UI.

L'invention a donc tout particulièrement pour objet les mélanges présentant les caractéristiques suivantes :
- un poids moléculaire moyen compris entre 1950 à 2150 Daltons.
- une activité anti-Xa compris entre 190 UI/mg et 410 UI/mg et une activité anti-IIa inférieure à 0,2 UI/mg.
- ils renferment de 30 à 60 % de fraction hexasaccharide contenant de 25 à 55 % de fraction ΔIIa-IIs-Is.

L'activité des mélanges d'oligosaccharide selon l'invention est obtenue par le biais d'un procédé très particulier qui est exposé ci-dessous. Il est bien connu de l'homme du métier que les caractéristiques physico-chimiques des mélanges de polysaccharides ainsi que l'activité qui en découle sont liées au procédé d'obtention (J. Med Chem 33(6) 1639-2093 (1990)).

Les mélanges d'oligosaccharides selon l'invention sont préparés par dépolymérisation d'un sel d'ammonium quaternaire de l'ester benzylique d'une héparine de très bas pois moléculaire (HTBPM) en milieu organique, cette (HTBPM) étant elle-même préparée selon l'enseignement des demandes de brevets WO 02/08295 et WO2004/033503. Il s'agit de manière générale de redépolymériser une héparine de très bas poids moléculaire qui elle même a été obtenue de manière spécifique par dépolymérisation de l'héparine estérifiée en présence d'une base forte, de préférence dans le dichlorométhane, et en présence d'un pourcentage d'eau inférieur à 3 %.

Les HTPBM utilisées comme matière première dans cette invention ont été préparées en particulier selon les procédés décrits dans les demandes de brevets WO 02/08295 et WO2004/033503.

Les HTBPM utilisées à titre de produit de départ ont notamment une activité aXa supérieures à 140 UI /mg une activité aIIa inférieure à 5 UI/mg et des masses moléculaires moyennes comprises entre 2000 et 3000 Daltons. La mesure des activités aXa est réalisée par rapport à une HTBPM standard d'activité mesurée à 158 UI/mg. L'activité de l'HTBPM standard est mesurée par rapport à l'étalon international standard d'héparine de bas poids moléculaire.

Les HTPBM de départ obtenues selon le procédé tel que décrit plus haut sont redépolymerisées au moyen d'une base organique forte de pka de préférence supérieur à 20 (propriétés de préférence apparentées à la famille des phosphazènes définies par exemple par R. Schwesinger et al, Angew. Chem. Int. Ed. Engl. 26, 1167-1169 (1987) ou R. Schwesinger et al, Angew. Chem. 105, 1420 (1993)). Ensuite on transforme le sel d'ammonium quaternaire de l'ester benzylique de l'HTBPM dépolymérisée en sel de sodium, saponifie les esters résiduels et éventuellement purifie le produit obtenu. Le schéma réactionnel suivant illustre la présente invention :

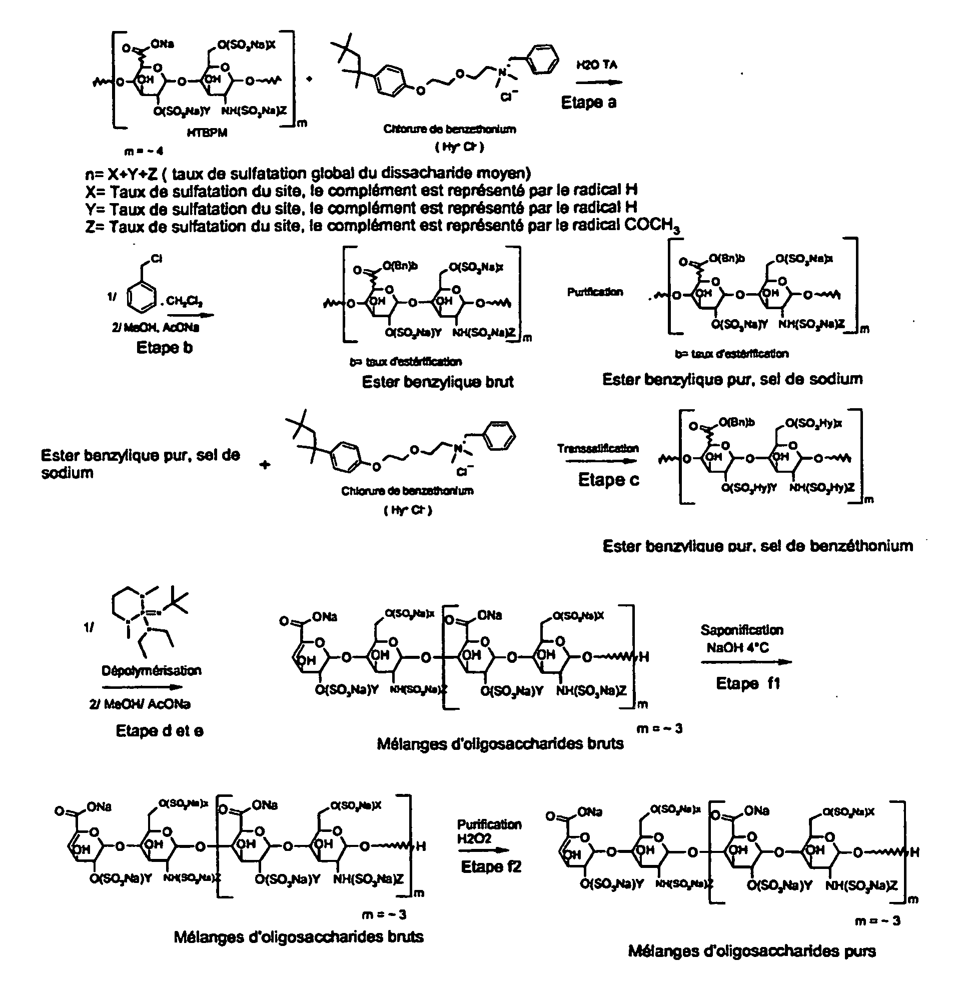

L'invention a donc également pour objet un procédé de préparation des mélanges d'oligosaccharides tels que définis plus haut **caractérisé en ce qu**'une héparine de très bas poids moléculaire, ayant une activité aXa supérieure à 140 UI/mg, une activité aIIa inférieure à 5 UI/mg et une masse moléculaire moyenne comprise entre 2000 et 3000 Daltons, subit les réactions chimiques suivantes :
a) Transalification par action de chlorure de benzéthonium pour obtenir l'héparinate de benzétonium,
b) Estérification de l'héparinate de benzéthonium obtenu par action de chlorure de benzyle, et traitement par une solution alcoolique d'acétate de sodium pour obtenir le sel de sodium de l'ester benzylique de l'héparine de très bas poids moléculaire,
c) Transalification de l'ester benzylique obtenu et obtention du sel d'ammonium quaternaire, de préférence en sel de benzéthonium, de cétyl pyrinium ou de cétyltriméthyl ammonium,
d) Dépolymérisation au moyen d'une base organique forte de pka de préférence supérieur à 20 afin d'obtenir une héparine de très bas poids moléculaire dépolymérisée,
e) Transformation du sel d'ammonium quaternaire de l'héparine de très bas poids moléculaire dépolymérisée en sel de sodium
f) Saponification des esters résiduels et éventuellement purification.

Dans la présente invention, on utilise tout particulièrement la forte sélectivité de la base phosphazène lors l'étape de dépolymérisation (étape d) pour enrichir de façon inattendue le mélange d'oligosaccharides en séquences affines à l'ATIII. De préférence, le rapport en mole base forte/ester est compris entre 0,2 et 5 et, tout particulièrement, entre 0,6 et 2.

Pour une selectivité optimale et une préservation maximale des séquences affines à l'ATIII, il est préférable de travailler à des teneurs en eau inférieures à 0,3 % lorsque l'on travaille avec 1 équivalent molaire de base phosphazène par rapport à l'ester benzylique de l'HTBTM, sel de benzéthonium.

Les bases de la famille des phosphazènes sont, de préférence, celles de formule : 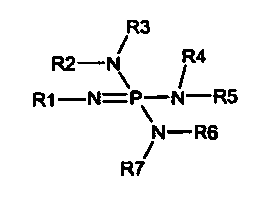 dans laquelle les radicaux R₁ à R₇, identiques ou différents, représentent des radicaux allyles linéaires, ramifiés ou cycliques contenant de 1 à 6 atomes de carbone R₃ et R₄ pouvant le cas échéant former avec le groupement -N-P-N- qui les porte un hétérocycle à 6 chaînons. En particulier l'invention a pour objet le procédé tel que défini plus haut **caractérisé en ce que** la base utilisée lors de l'étape d) de dépolymérisation est le 2-tert-butylimino-2-diethylamino-1,3-diméthylperhydro-1,3,2-diaza-phosphorine (nomenclature officielle : 1,3,2-diazaphosphorin-2-amine, 2-[(1,1-dimethylethyl)imino]-N,N-diéthyl-1,2,2,2,3,5,6-octahydro-1,3-diméthyl).

La réaction de l'étape de transalification a) est réalisée de préférence par action du chlorure de benzéthonium en excès, sur l'HTBPM sodique, à une température voisine de 15 à 25°C. De manière avantageuse, le rapport molaire sel/héparine sodique est compris entre 2,5 et 3,5.

L'estérification de l'étape b) s'effectue de préférence au sein d'un solvant organique chloré (tel que le chloroforme ou le dichlorométhane), à une température comprise entre 25 et 45°C et, de préférence entre 30 et 40°C. L'ester sous forme de sel de sodium est ensuite récupéré par précipitation au moyen d'acétate de sodium à 10 % en poids dans un alcool tel que le méthanol. On emploie généralement 1 à 1,2 volume d'alcool par volume de milieu réactionnel. La quantité de chlorure de benzyle et le temps de réaction sont adaptés pour obtenir un taux d'estérification compris entre 40 et 100 % et, de préférence, entre 70 et 90 %. De façon préférentielle, on utilise 0,5 à 1,5 parties en poids du chlorure de benzyle pour 1 partie en poids du sel de benzéthonium de l'héparine. De même, de façon préférentielle, le temps de réaction sera compris entre 10 et 35 h.

Par conséquent, le procédé selon l'invention met en oeuvre un taux d'estérification du sel d'ammonium quaternaire de l'ester benzylique de l'héparine compris entre 40 et 100 % et, de préférence, entre 70 et 90 %.

La transformation du sel d'ammonium quaternaire de l'ester benzylique de l'héparine dépolymérisée en sel de sodium est effectuée, généralement, par traitement du milieu réactionnel avec une solution alcoolique d'acétate de sodium et, de préférence avec une solution à 10 % d'acétate de sodium dans le méthanol (poids/volume), à une température comprise entre 15 et 25°C.

L'équivalent en poids d'acétate ajoutée est préférentiellement 3 fois supérieur à la masse de sel d'ammonium quaternaire de l'ester benzylique de l'héparine engagé par la suite dans la réaction de dépolymérisation. Le sel d'ammonium quaternaire de l'ester benzylique de l'HTBPM obtenue est de préférence le sel de benzéthonium, de cétylpyridinium ou de cétyltriméthyl ammonium.

La transalification de l'étape c) s'effectue au moyen d'un chlorure d'ammonium quaternaire et, de préférence, au moyen de chlorure de benzéthonium, de chlorure de cétylpyridinium ou de chlorure de cétyltriméthyl ammonium, en milieu aqueux, à une température comprise entre 10 et 25°C. De manière avantageuse, le rapport en mole chlorure d'ammonium quaternaire/sel de sodium de l'ester benzylique de l'héparine est compris entre 2,5 et 3,5.

La saponification s'effectue généralement au moyen d'un hydroxyde de métal alcalin tel que la soude, la potasse, l'hydroxyde de lithium, en milieu aqueux, à une température comprise entre 0 et 20°C et de préférence 0 et 10°C. On utilisera de façon générale de 1 à 5 équivalents molaires d'hydroxyde de métal alcalin. De préférence, la saponification sera réalisée en présence de 1 à 2 équivalents molaires d'hydroxyde de métal alcalin.

Le produit final peut éventuellement être purifié par toute méthode connue de purification des héparines dépolymérisées (par exemple EP 0037319B1). De préférence, on purifie au moyen de peroxyde d'hydrogène, en milieu aqueux, à une température de 10 à 50°C. De préférence, cette opération sera réalisée entre 20 et 40°C.

Les mélanges selon l'invention sous forme de sel de sodium, peuvent être transformés en un autre sel d'un métal alcalin ou alcalinoterreux. On passe éventuellement d'un sel à l'autre en utilisant la méthode décrite dans le brevet FR 73 13 580.

La présente invention permet notamment un fort enrichissement en hexasaccharide ΔIIa-IIs-Is. Lorsque l'on re-dépolymérise une héparine de bas poids moléculaire par le procédé qui l'a généré, il est bien connu de l'homme du métier que l'activité anti-Xa du produit obtenu décroît fortement jusqu'à être nulle. Dans le cas où l'on utilise les procédés permettant l'obtention de l'Enoxaparine, la Fraxiparine, la Fragmine, l'Innohep (ou Logiparin), le Normiflo, l'Embollex (ou Sandoparin), le Fluxum (ou Minidalton), la Clivarine et l'Hibor, on peut observer ce phénomène si on re-dépolymérise ces HBPM avec leur procédé d'origine. Ceci est la conséquence de la faible sélectivité de ces procédés à vis de la préservation des sites ATIII.

Dans la présente invention, si l'on utilise comme matière première, une HTBPM issue du procédé de dépolymérisation phosphazène, c'est exactement le phénomène inverse qui se produit. L'activité aXa du mélange d'oligosaccharide augmente et dépasse même celle de l'héparine qui à servi à préparer l'HTBPM. Ceci est la conséquence probable de la sélectivité remarquable des bases phosphazènes sur la préservation des séquences affines à l'ATIII.

Cette caractéristique du procédé s'observe aussi au travers des masses moléculaires moyennes du mélange d'oligosaccharides obtenus. A titre d'exemple, lorsque l'on dépolymérise une HTBPM de masse moléculaire moyenne de 2400 Daltons, on obtient un mélange d'oligosaccharides de masse moléculaire moyenne de 2000 Da. On constate que les séquences affines à l'ATIII (hexasaccharides et octasaccharides) sont préservées de l'action de la base phosphazène, ce qui entraîne la destruction et l'élimination des autres séquences, et par voie de conséquence, une masse moléculaire moyenne qui va tendre vers la masse moleculaire moyenne de l'entité non dépolymerisable ; c'est à dire l'hexasaccharide ΔIIa-IIs-Is (1834 g/mol). Il est à souligner que dans l'étape de dépolymerisation de l'héparine avec une base phosphazène, on passe d'une masse moléculaire moyenne d'environ 15000 Da à environ 2400 Da.

A titre de méthode alternative, le procédé selon l'invention permettant d'augmenter l'activité et la sélectivité envers le facteur Xa est également applicable aux heparines de bas poids moléculaire en général. A titre d'exemple, on citera par exemple l'Enoxaparine, la Fraxiparine, la Fragmine, l'Innohep (ou Logiparin), le Normiflo, l'Embollex (ou Sandoparin), le Fluxum (ou Minidalton), la Clivarine et l'Hibor. Il peut également s'agir de certaines héparines de très bas poids moléculaire telles que décrites dans US 6,384,021 (2000 - 4000 Da) ou WO 02/08295 (1500 à 3000 Da) ayant une activité anti-Xa présentant une activité and-Xa inférieure à 140 UI/mg. (notamment comprise entre 100 et 140 UI/mg)

Cette caractéristique du procédé se traduit par l'obtention d'activités anti-Xa inattendues au regard du poids moléculaire moyen des mélanges d'oligosaccharides (190 UI/mg < aXa < 450 UI/mg ; 1800 Da < PM < 2400Da).

Selon un mode particulier de l'invention, la sélectivité vis à vis du facteur Xa du mélanges d'oligosaccharides peut être encore augmentée en éliminant les fractions disaccharides et tétrasaccharides (fractions ne liant pas spécifiquement à l'ATIII). Le mélange est dans ce cas chromatographié sur des colonnes remplies de gel de type polyacrylamide agarose ou un gel de polyacrylamide. Le mélange est élué par une solution d'hydrogénocarbonate de sodium. De préférence, la solution d'hydrogénocarbonate de sodium est une solution de 0.1 mol/ 1 à 1 mol/l. Encore plus préférentiellement, la séparation est réalisée a une concentration de 1 mole/l. La détection est réalisée par spectrométrie UV (254nm). Après élimination des fractions disaccharides et tetrasaccharides, le mélange d'oligosaccharides en solution dans l'hydrogénocarbonate de sodium est neutralisée par de l'acide acétique glacial. La solution est ensuite concentrée sous pression réduite de façon à obtenir une concentration en acétate de sodium supérieure à 20 % en poids. Le mélange d'oligosaccharides est précipitée par addition de 3 à 5 volumes de méthanol. Le mélange d'oligosaccharide hautement affin est récupéré par filtration. Si cela s'avère nécessaire, il peut être purifié par désalage sur une colonne appropriée. L'exemple 6 illustre cette méthode alternative et permet d'obtenir des héparines de très bas poids moléculaire dont l'activité anti-Xa est supérieure à 400 UI/mg.

L'invention a donc également pour objet un procédé tel que définit plus haut de préparation de mélanges d'oligosaccharides possédant une sélectivité vis-à-vis du facteur Xa du mélanges d'oligosaccharides augmentée **caractérisé en ce qu**'en outre on élimine les fractions disaccharides et tétrasaccharides par chromatographie notamment sur des colonnes remplies de gel de type polyacrylamide agarose.

Les mélanges selon l'invention peuvent être utilisés à titre de médicaments.

Les mélanges d'oligosaccharides de la présente invention peuvent être utilisés comme agents antithrombotiques. En particulier, ils sont utiles pour le traitement ou la prévention des thromboses veineuses et artérielles, la thrombose veineuse profonde, l'embolie pulmonaire, l'angor instable, l'infarctus du myocarde, l'ischémie cardiaque, les maladies occlusives des artères périphériques et la fibrillation auriculaire. Ils sont également utiles dans la prévention et le traitement de la prolifération des cellules musculaires lisses l'athérosclérose et l'artériosclérose, pour le traitement et la prévention du cancer par la modulation de l'angiogénèse et des facteurs de croissance, et pour le traitement et la prévention des désordres diabétiques tel que les rétinopathies et néphropathies diabétiques.

La présente invention concerne également les compositions pharmaceutiques contenant comme principe actif un mélange de formule (I) éventuellement en association avec un ou plusieurs excipients inertes.

Les compositions pharmaceutiques sont par exemple des solutions injectables par voie sous-cutanée ou intraveineuse. D'autres compositions pharmaceutiques selon l'invention sont également mises en oeuvre pour une administration par voie pulmonaire (inhalation) ou par voie orale.

La posologie peut varier en fonction de l'âge, du poids et de l'état de santé du patient. Pour un adulte, elle est en général comprise entre 20 et 100 mg par jour par voie intramusculaire ou sous-cutanée.

Les exemples suivants illustrent l'invention sans toutefois la limiter.

### Préparation 1 : Obtention d'une héparine de très bas poids moléculaire de départ présentant une activité aXa égale à 158,8 IU/mg

L'héparine de très bas poids moléculaire (HTBPM) utilisée à titre de produit de départ pour l'exemple 1 est préparée selon la demande de brevet WO2004/033503 à partir d'héparine de sodium en mettant en oeuvre les étapes a à f telles que définies plus haut, l'étape de dépolymérisation étant effectuée en présence de 2-tert-butylimino-2-diethylamino-1,3-diméthylperhydro-1,3,2-diaza-phosphorine en présence d'un pourcentage d'eau inférieur à 0,6 %.

### Caractéristiques de l'héparine de très bas poids moléculaire obtenue

Les caractéristiques de l'héparine dépolymérisée ainsi obtenue sont les suivantes :
Poids moléculaire moyen : 2400 Daltons
Activité anti-Xa : 158,8 UI/mg
Activité anti-IIa : 3,1 UI/mg
Rapport activité anti-Xa/activité anti-IIa :51

### Préparation 2 : Obtention d'une héparine de très bas poids moléculaire de départ présentant une activité aXa égale à 158 IU/mg

L'héparine de très bas poids moléculaire (HTBPM) utilisée à titre de produit de départ pour les exemples 2, 3, 4, 5 est préparée selon la demande de brevet WO2004/033503 à partir d'héparine de sodium en mettant en oeuvre les étapes a à f telles que définies plus haut, l'étape de dépolymérisation étant effectuée en présence de 2-tert-butylimino-2-diethylamino-l,3-diméthylperhydro-1,3,2-diaza-phosphorine en présence d'un pourcentage d'eau inférieur à 0,6 %.

### Caractéristiques de l'héparine de très bas poids moléculaire obtenue

Les caractéristiques de l'héparine dépolymérisée ainsi obtenue sont les suivantes :
Poids moléculaire moyen : 2450 Daltons
Activité anti-Xa : 158 UI/mg
Activité anti-IIa : 2,1 UI/mg
Rapport activité anti-Xa/activité anti-IIa :75

### Préparation 3 : HTBPM, sel de benzéthonium

Trans-salification de l'HTBPM en sel de benzéthonium (correspondant à l'étape a) du procédé) :
12,53 g (20,7 mmoles) d'HTBPM sel de sodium obtenu selon la préparation 1 sont chargés dans un erlenmeyer A de 500 ml et solubilisés dans 85 ml d'eau (solution jaune).
31,62 g (70,5 mmoles) de chlorure de benzéthonium sont chargés dans un erlenmeyer B de 100 ml avec 250 ml d'eau (solution incolore).

Le contenu de B est coulé dans A et le mélange est agité environ 1 h à température ambiante. On laisse sédimenter pendant environ 1 h. Le surnageant est écarté puis remplacé par le même volume de d'eau (250 ml). On agite pendant environ 15 minutes et laisse sédimenter approximativement 30 minutes. Le surnageant est écarté puis remplacé par le même volume d'eau (250 ml). Le mélange est agité environ 15 minutes puis filtré. Le gâteau est lavé par 3 fois 200 ml d'eau. Le solide beige humide est essoré puis séché à 80°C pendant environ 18 h en étuve sous pression réduite (6 kPa). On obtient 35,56 g d'HTBPM sel de benzéthonium.

Le rendement obtenu est de 89 %.

### Préparation 4

Trans-salification de l'HTBPM en sel de benzéthonium ( correspondant à l'étape a) du procédé) :
17.93 g (30,2mmoles) d'HTBPM sel de sodium obtenu selon la préparation 2 sont chargés dans un erlenmeyer A de 1 1 et solubilisés dans 120 ml d'eau (solution jaune).
45 g (0,1 moles) de chlorure de benzéthonium sont chargés dans un erlenmeyer B de 500 ml avec 360 ml d'eau (solution incolore).

Le contenu de B est coulé dans A et le mélange est agité environ 1 h à température ambiante. On laisse sédimenter pendant environ 1 h. Le surnageant est écarté puis remplacé par le même volume de d'eau (500 ml). On agite pendant environ 15 minutes et laisse sédimenter approximativement 30 minutes. Le surnageant est écarté puis remplacé par le même volume d'eau (500 ml). Le mélange est agité environ 15 minutes puis filtré. Le gâteau est lavé par 3 fois 200 ml d'eau. Le solide beige humide est essoré puis séché à 80°C pendant environ 48 h en étuve sous pression réduite (6 kPa). On obtient 49,5g d'HTHPM sel de benzéthonium.

Le rendement obtenu est de 87 %.

### Exemple 1 :

Héparine de très bas poids moléculaire (HTBPM) obtenue par le procédé selon l'invention comprenant une étape d'estérification à 77 % et une étape de dépolymerization avec une base dérivé de phosphazene en milieu anhydre

### . Estérification de l'HTBPM (étape b du procédé) :

35,39 g (18,3 mmoles) d'HTBPM sel de benzéthonium obtenue selon la préparation 3 (avec une teneur en eau de 0.20 %) sont solubilisés dans 183,3 g de dichlorométhane sec et chargés dans un tricol de 500 ml. 29,5 ml (25,7 mmoles) de chlorure de benzyle sont ajoutés à la température de 30°C. Le taux d'estérification est de 77 % après environ 23 h de réaction à 30°C. Après refroidissement à température ambiante (22±3°C), le mélange réactionnel est versé sur 490 ml d'une solution d'acétate de sodium à 10 % dans le méthanol. Le mélange est agité pendant environ 1 h puis on laisse sédimenter approximativement 1 h. Le surnageant est écarté puis remplacé par le même volume de méthanol (250 ml). On agite approximativement 30 minutes puis on laisse sédimenter environ 45 minutes. Le surnageant est écarté puis remplacé par le même volume de méthanol (250 ml). On laisse sédimenter environ 16 h. Le surnageant est écarté puis remplacé par le même volume de méthanol (350 ml). On agite pendant environ 5 minutes et la suspension est filtrée. Le gâteau est lavé par 2 fois 50 ml de méthanol, essoré puis séché à 40°C sous pression réduite (6 kPa) pendant environ 18 h. On obtient 34,48 g d'HTBPM ester de benzyle sel de sodium brut avec un taux d'estérification de 77 %.

### . Purification de l'HTBPM ester de benzyle sel de sodium (étape b) du procédé).

Les 34,48 g d'HTBPM ester de benzyle sel de sodium brut sont solubilisés dans 350 ml d'une solution aqueuse de NaCl à 10 %. La solution est coulée sur 1,57 1 de méthanol. La suspension est agitée pendant environ 40 minutes puis on laisse sédimenter pendant environ 16 h. Le surnageant est écarté et remplacé par le même volume de méthanol (1,5 1). On agite environ 1 h et on laisse sédimenter pendant environ 1,5 h. Le surnageant est écarté et remplacé par le même volume de méthanol (1,2 1). On agite approximativement 15 minutes puis on filtre. Le gâteau est lavé par 3 fois 50 ml de méthanol. Le solide humide blanc est essoré puis séché à 40°C sous pression réduite (6 kPa) pendant environ 18 h. On obtient 6,07 g d'HTBPM ester de benzyle sel de sodium.

Le rendement de l'estérification est de 50 %.

### . Trans-salification de l'HTBPM ester de benzyle, sel de sodium en sel de benzéthonium (étape c) du procédé):

6 g (9,14 mmole) d'HTBPM ester de benzyle sel de sodium sont solubilisés avec 40 ml d'eau dans un erlenmeyer A de 250 ml.

Pendant ce temps, 13,93 g (31 mmoles) de chlorure de benzéthonium sont chargés avec 110 ml d'eau dans un erlenmeyer B de 250 ml.

Le contenu de B est coulé dans A. La suspension est agitée environ 1 h à température ambiante (22±3°C) puis laissée sédimenter 1 h. Le surnageant est écarté et remplacé par le même volume d'eau (140 ml). On agite pendant environ 15 minutes et on laisse sédimenter 1 h. Le surnageant est écarté et remplacé par le même volume d'eau (140 ml). On agite approximativement 15 minutes et laisse sédimenter de l'ordre de 30 minutes. Le surnageant est écarté et remplacé par le même volume d'eau (140 ml). On agite environ 5 minutes puis on filtre. Le gâteau est lavé par 3 fois 50 ml d'eau, essoré puis séché à 80°C sous pression réduite (6 kPa) pendant environ 18 h. On obtient 17,43 g d'HTBPM ester de benzyle, sel de benzéthonium.

Le rendement est de 100 %.

### . Dépolymérisation de l'HTBPM ester de benzyle, sel de benzéthonium en milieu anhydre : Teneur en eau non détectable < 0.01% (étape d) du procédé)

17,43 g (9,14 mmole) d'HTBPM selon la préparation 2 sont chargés dans un tricol de 250 ml avec 122 ml de dichlorométhane sec. 17,4 g de tamis moléculaire 4 Å sont additionnés. L'ensemble est agité pendant environ 18h à température ambiante (22±3°C) sous atmosphère d'argon.

Le tamis est séparé du mélange par transfert de la solution dans un tricol de 250 ml. On ajoute 2,64 ml (9,14 mmole) de 2-tert-butylimino-2-diethylamino-1 ,3-diméthylperhydro-1,3,2-diaza-phosphorine et on agite pendant 24 h à 22±3°C sous atmosphère d'argon.

### . Transformation du sel d'ammonium quaternaire en sel de sodium (étape e) du procédé)

Pendant ce temps, 730 ml d'une solution méthanolique d'acétate de sodium à 10 % est préparée dans un erlenmeyer de 21. 8,71 g de célite Hyflo supercel sont ajoutés à la solution. Le mélange réactionnel est coulé dans la solution méthanolique en maintenant la température à environ 4°C. La suspension est agitée pendant environ 15 minutes à cette température. On laisse sédimenter approximativement 45 minutes à température ambiante puis le surnageant est écarté puis remplacé par la même quantité de méthanol (450 ml). On agite 15 minutes et on laisse sédimenter approximativement 45 minutes. Le surnageant est à nouveau écarté et remplacé par la même quantité de méthanol (420 ml). On agite pendant environ 15 minutes puis on filtre sur verre fritté n° 3. Le gâteau est lavé par 2 fois 70 ml de méthanol, essoré puis séché pendant environ 18 h à 50°C sous pression réduite (6 kPa). On obtient 4,35 g d'HTBPM dépolymérisée brute (sel de sodium) dans la célite (8,71 g).

Le rendement est de 72,5 %.

### . Saponification de l'HTBPM dépolymerisée brute, sel de sodium (étape f1) du procédé) :

4,35 g (6,63 mmole) d'HTBPM dépolymérisée brute (sel de sodium) dans la célite est solubilisée dans 46 ml d'eau puis filtrée sur verre fritté n° 3. La célite est rinçée par 2 portions de 30 ml d'eau. Le filtrat est chargé dans un erlenmmeyer de 500 ml. On introduit 823 µl (9,94 mmole) de lessive de soude à 35 % à une température voisine de 4 °C. On agite pendant environ 3 h à cette température. Le milieu est neutralisé par addition d'une solution d'HCl 1N puis on ajoute 11,5 g de NaCl et 80 ml de méthanol. Après approximativement 15 minutes d'agitation, 210 ml de méthanol sont ajoutés. La suspension est agitée pendant environ 1 h puis laissée sédimenter 30 minutes. Le surnageant est écarté, remplacé par la même quantité de méthanol (230 ml). On agite pendant environ 15 minutes et on laisse sédimenter 30 minutes. Le surnageant est écarté et remplacé par la même quantité de méthanol (210 ml). On agite approximativement 15 minutes puis on filtre. Le gâteau est lavé par 2 fois 9 ml de méthanol, essoré puis séché pendant environ 18 h à 50°C sous pression réduite (6 kPa). On obtient 2,95 g d'HTBPM dépolymérisée brute (sel de sodium).

Le rendement est de 73,7 %.

### f) Purification de l' HTBPM dépolymérisée brute -sel de sodium- (étape f2 du procédé) :

1,5 g d' HTBPM dépolymérisée brute, sel de sodium sont chargés dans un tricol de 50 ml avec 16 ml d'eau. La solution est portée pendant environ10 minutes à 40 °C. Le pH est amené à environ 9,7 par addition de soude 0,1 N. La solution est filtrée sur membrane 0,45 µm puis 84 µl d'une solution aqueuse de peroxyde d'hydrogène à 30 % sont ajoutés. Le mélange est agité 2 h à température ambiante en maintenant le pH constant à 9,7 ± 0,1 par addition de soude 0,1 N. Le mélange réactionnel est ensuite neutralisé par HCl 0,1 N puis 2 g de NaCl sont additionnés. Après environ 10 minutes d'agitation, la solution est filtrée sur membrane 0,45 µm. 14 ml de méthanol sont coulés à une température voisine de 4 °C. La solution est agitée approximativement pendant 15 minutes à température ambiante. On ajoute ensuite 36 ml de méthanol et la suspension est agitée pendant environ 1 h. L'agitation est alors arrêtée et on laisse sédimenter pendant environ 30 minutes. Le surnageant est ensuite prélevé puis écarté (40 ml). Sur le précipité sédimenté, on ajoute 40 ml de méthanol et on agite pendant environ 10 minutes. On laisse re-sédimenter le précipité approximativement 30 minutes. Le surnageant est prélevé et écarté (45 ml). 45 ml de méthanol sont ajoutés et le précipité en suspension est ensuite filtré. Le gâteau blanc obtenu est ensuite lavé par 2 portions de 3 ml de méthanol. Le solide humide est essoré puis séché sous pression réduite (6 kPa), à une température voisine de50 °C. Après environ 18 heures de séchage, on obtient 1.303 g d'HTBPM dépolymérisée pure (sel de sodium).

Le rendement obtenu est de 86,8 %.

### g) Caractéristique de l'HTBPM dépolymérisée ainsi obtenue

Poids moléculaire moyen : 1950 Daltons
Indice de polydispersité : 1.1
Activité anti-Xa : 283 U/mg
Activité aIIa : non détectable (< 0.2 U/mg)

### Exemple 2 :

Héparine de très bas poids moléculaire (HTBPM) obtenue par le procédé selon l'invention comprenant une étape d'estérification à 49 % et une étape de dépolymerization avec une base dérivé de phosphazene en milieu anhydre

### . Estérification de l'HTBPM (étape b du procédé) :

13,29 g (7,6 mmoles) d' HTBPM sel de benzéthonium obtenue selon la préparation 4 sont solubilisés dans 70,43 g de dichlorométhane anhydre et chargés dans un tricol de 100 ml (La teneur en eau du milieu réactionnel est de 0,073 %). 12,3 ml (107 mmoles) de chlorure de benzyle sont ajoutés à la température de 30°C. Le taux d'estérification est de 49 % après environ 7 h de réaction à 30°C. Après refroidissement, le mélange réactionnel est versé sur 160 ml d'une solution à 12 % d'acétate de sodium dans le méthanol. Le mélange est agité 1 h à l'ambiante puis laissé sédimenter pendant environ 16 h. Le surnageant est écarté puis remplacé par le même volume de méthanol (100 ml). On agite environ 1 h et on laisse sédimenter environ 1 h. Le surnageant est à nouveau écarté et remplacé par le même volume de méthanol (100 ml). On agite environ 5 minutes puis on filtre. Le gâteau est lavé par 2 x 40 ml de méthanol, essoré puis séché en étuve à 40 °C sous pression réduite (6 kPa) pendant environ 18 h. On obtient 3,90 g d' HTBPM ester de benzyle sel de sodium brut avec un taux d'estérification de 49 %.

### . Purification de l'HTBPM ester de benzyle (estérifié à 49 %) sel de sodium (étape b du procédé).

3,90 g d'HTBPM ester de benzyle sel de sodium brut sont solubilisés dans 39 ml d'une solution aqueuse de NaCl à 10 %. La solution est coulée sur 176 ml de méthanol. La suspension est agitée pendant environ 15 minutes puis on laisse sédimenter 2 h. Le mélange est filtré. Le gâteau est remis en suspension dans 175 ml de méthanol et agité pendant 10 minutes. On filtre et le gâteau est lavé par 2 portions de 10 ml de méthanol. Le solide humide blanc est essoré, séché en étuve à 40°C sous pression réduite (6 kPa) pendant environ 18 h. On obtient 2,62 g d'HTBPM ester de benzyle sel de sodium.

Le rendement global de la phase estérification est de 57,3 %.

### . Trans-salification de l'HTBPM ester de benzyle en sel de benzéthonium (étape c) du procédé) :

2,62 g (4,37 mmoles) d' HTBPM ester de benzyle sel de sodium sont solubilisés dans 20 ml d'eau (erlenmeyer « A »). Pendant ce temps, 5,92 g (13,2 mmoles) de chlorure de benzéthonium sont chargés avec 60 ml d'eau dans un erlenmeyer « B ». Le contenu de « B » est coulé dans « A ». La suspension est agitée pendant environ1 h à température ambiante puis laissée sédimenter 1 h. Le surnageant est écarté et remplacé par le même volume d'eau (70 ml). On agite pendant environ 15 minutes et on laisse sédimenter 1 h. Le surnageant est écarté et remplacé par le même volume d'eau (70 ml). On agite encore environ 5 minutes et on filtre. Le gâteau est lavé par 3 portions de 50 ml d'eau, essoré puis séché en étuve à 80°C sous pression réduite (6 kPa) pendant environ 18 h. On obtient 6,85 g d' HTBPM ester de benzyle sel de benzéthonium.

Le rendement est de 99 %. La teneur en eau du sel de benzéthonium est de 0.6 %.

### . Dépolymérisation de l'HTBPM ester de benzyle, sel de benzéthonium :

6,80 g (4,3 mmoles) d'HTBPM sont chargés dans un tricol de 100 ml avec 54 ml de dichlorométhane sec. Le mélange est porté à 30°C puis agité jusqu'à dissolution complète. Le teneur en eau estimée du mélange réactionnel est d'environ 0,05 %. On ajoute 1,25 ml (4,3 mmoles) de 2-tert-butylimino-2-diethylamino-1 ,3-diméthylperhydro-1,3,2-diaza-phosphorine et on agite 24 h à 30°C sous atmosphère inerte.

### . Transformation du sel d'ammonium quaternaire en sel de sodium (étape e) du procédé)

Pendant ce temps, 270 ml d'une solution méthanolique d'acétate de sodium à 10 % sont préparés dans un erlenmeyer de 1 1. Le mélange réactionnel est coulé dans la solution méthanolique en maintenant la température à environ 4°C. La suspension est agitée environ1 h à température ambiante. On laisse sédimenter pendant 1 h. Le surnageant est écarté puis remplacé par la même quantité de méthanol (165 ml). On agite environ 1 h et laisse sédimenter 1 h. Le surnageant est à nouveau écarté et remplacé par la même quantité de méthanol (170 ml). On agite pendant environ 15 minutes et on filtre. Le gâteau est lavé par 3 portions 40 ml de méthanol, essoré puis séché pendant environ 18 h en étuve à 50°C sous pression réduite (6 kPa). On obtient 2,29 g d'HTBPM dépolymérisée brute, sel de sodium.

Le rendement obtenu est de 89 %.

### . Saponification de l'HTBPM brute, sel de sodium (étape f1 du procédé) :

2,29 g (3,8 mmoles) d' HTBPM dépolymérisée brute, sel de sodium sont solubilisés dans 23 ml d'eau. La solution est filtrée sur membrane de 0,8 µm puis chargée dans un tricol de 100 ml. On introduit 575 µl (5,73 mmoles) de lessive de soude à 30 % à une température voisine de 3 °C. On agite pendant environ 2 h à cette température.

La moitiée du mélange réactionnel est neutralisé par addition de l'acide acétique glacial et on ajoute ensuite 367 mg d'acétate de sodium solide et 13 ml de méthanol. La solution est agité environ 15 minutes puis 65 ml de méthanol sont ajoutés. La suspension obtenue est agitée environ 30 minutes puis on laisse sédimenter environ 16 heures. Le surnageant est écarté et remplacé par la même quantité de méthanol (36 ml). On agite encore approximativement 30 minutes et laisse sédimenter environ 30 minutes. Le surnageant est écarté et remplacé par la même quantité de méthanol (16 ml). On agite environ 15 minutes et on filtre sur membrane de 0,22 µm. Le gâteau est lavé par 2 fois 5 ml de méthanol, essoré puis séché sous pression réduite (6 kPa) pendant environ 18 h en étuve à 50°C. On obtient 563 mg d'HTBPM dépolymérisée brute (sel de sodium).

Le rendement est de 52,6 %.

### . Purification de l'HTBPM dépolymérisée brute (sel de sodium) précipitée par AcONa (étape f2 du procédé) :

560 mg d'HTBMP dépolymérisée brute (sel de sodium) sont chargés dans un tricol de 10 ml avec 5,6 ml d'eau. La solution brune est portée 10 minutes à 40 °C. Le pH est amené à 9,7 par addition de soude 0,1 N. La solution est filtrée sur membrane 0,45 µm, 28 µl d'une solution aqueuse de peroxyde d'hydrogène à 30 % sont ajoutés. Le mélange est agité 2 h à température ambiante en maintenant le pH constant à 9,5 ± 0,1 par addition de soude 0,1 N. Le mélange réactionnel est neutralisé par HCl 0,1N, 620 mg de NaCl sont additionnés. Après 10 minutes d'agitation, la solution est filtrée sur membrane 0,45 µm. 4,35 ml de méthanol sont coulés à une température voisine de 4°C. La solution est agitée 15 minutes à température ambiante. 11,2 ml de méthanol sont ajoutés. La suspension est agitée 1 h. L'agitation est alors arrêtée et laisse sédimenter pendant 1 h. Le surnageant est ensuite prélevé puis écarté (13,5 ml). Sur le précipité sédimenté, on ajoute 13,5 ml de méthanol et on agite pendant 15 minutes. On laisse re-sédimenter le précipité 30 minutes environ. Le surnageant est prélevé et écarté (13 ml). 13 ml de méthanol sont ajoutés et le précipité en suspension est ensuite filtré. Le gâteau blanc obtenu est ensuite lavé par 2 portions de 5 ml de méthanol. Le solide humide est essoré puis séché sous pression réduite (6 kPa), à une température voisine de 50°C. Après 18 heures de séchage, on obtient 376 mg d'HTBPM dépolymérisée pure (sel de sodium). Le rendement obtenu est de 67 %.

### Caractéristique de l'HTBPM dépolymérisée ainsi obtenue

Activité anti-Xa : 191UI/mg
Poids moléculaire moyen: 2100 Da

### Exemple 3 :

Héparine de très bas poids moléculaire (HTBPM) obtenue par le procédé selon l'invention comprenant une étape d'estérification à 73 % et une étape de dépolymerization avec une base dérivé de phosphazène en milieu anhydre

### . Estérification de l'HTBPM (étape b du procédé) :

13,7 g (7,3 mmoles) d'HTBPM sel de benzéthonium obtenue selon la préparation 4 sont solubilisés dans 73,67 g de dichlorométhane anhydre et chargés dans un tricol de 100 ml. (La teneur en eau du milieu réactionnel est dosée à 0,23 %). 13 ml (113 mmoles) de chlorure de benzyle sont ajoutés à la température de 30°C. Le taux d'estérification est de 73 % après environ 20 h de réaction à 30 °C. Après refroidissement à température ambiante, le mélange réactionnel est versé sur 210 ml d'une solution à 12 % d'acétate de sodium dans le méthanol. Le mélange est agité 30 minutes à temperature ambiante puis on laisse sédimenter pendant environ 1,5 h. Le surnageant est écarté puis remplacé par le même volume de méthanol (140 ml). On agite 15 minutes et la suspension est filtrée. Le gâteau est lavé par 2 portions de 100 ml de méthanol, essoré puis séché approximativement 18 h en étuve à 40°C sous pression réduite (6 kPa). On obtient 13,3 g d'HTBPM ester de benzyle sel de sodium brut avec un taux d'estérification de 73 %.

### . Purification de l'HTBPM ester de benzyle (estérifié à 73%), sel de sodium (étape b du procédé) .

Les 13,3 g d'HTBPM ester de benzyle sel de sodium brut sont solubilisés dans 133 ml d'une solution aqueuse de NaCl à 10 %. La solution est coulée sur 600 ml de méthanol. La suspension est agitée pendant environ 15 minutes puis laisse sédimenter approximativement 1 h. Le surnageant est écarté puis remplacé par le même volume de méthanol (400 ml). On agite pendant environ 5 minutes puis on filtre. Le gâteau est lavé par 3 fois 100 ml de méthanol. Le solide humide blanc est essoré puis séché approximativement 18 h en étuve à 40°C sous pression réduite (6 kPa). On obtient 2,33 g d'HTBPM ester de benzyle sel de sodium.

Le rendement d'estérification est de 49,6 %.

### . Trans-salifcation de l'HTBPM ester de benzyle, sel de benzéthonium (étape c du procédé) :

2,27 g (3,53 mmoles) d'HTBPM ester de benzyle sel de sodium sont solubilisés avec 15 ml d'eau dans un erlenmeyer « A » de 100 ml. Pendant ce temps 5,22 g (11,6 mmoles) de chlorure de benzéthonium sont chargés avec 55 ml d'eau dans un erlenmeyer « B » de 100 ml.

Le contenu de « B » est coulé dans « A ». La suspension est agitée environ 1 h à température ambiante puis on laisse sédimenter approximativement 1 h. Le surnageant est écarté et remplacé par le même volume d'eau (50 ml). On agite environ 15 minutes et laisse sédimenter approximativement 1 h. Le surnageant est écarté et remplacé par le même volume d'eau (50 ml). On agite encore 5 minutes puis on filtre. Le gâteau est lavé par 3 portions de 50 ml d'eau, essoré puis séché pendant environ 18h en étuve à 80°C sous pression réduite (6 kPa). On obtient 5,67 g d'HTBPM ester de benzyle sel de benzéthonium.

Le rendement obtenu est de 98 %. La teneur en eau du produit obtenu est de 1 %

### . Dépolymérisation de l'HTBPM ester de benzyle, sel de benzéthonium (étape d du procédé) :

5,45 g (3,3 mmoles) d'HTBPM sont chargés dans un tricol de 100 ml avec 40 ml de dichlorométhane sec. La teneur en eau estimée du mélange est d'environ 0.1%. Le mélange est porté à 30°C. On ajoute 958 µl (3,3 mmoles) de 2-tert-butylimino-2-diethylamino-1 ,3-diméthylperhydro-1,3,2-diaza-phosphorine et agite 24 h à 30°C sous atmosphère d'argon.

### . Transformation du sel d'ammonium quaternaire en sel de sodium (étape e) du procédé)

Pendant ce temps, 200 ml d'une solution méthanolique d'acétate de sodium à 10 % est préparée dans un erlenmeyer de 500 ml. Le mélange réactionnel est coulé dans la solution méthanolique en maintenant la température à environ 4°C. La suspension est agitée pendant environ 1 h à température ambiante. On laisse sédimenter approximativement 1 h. Le surnageant est écarté puis remplacé par la même quantité de méthanol (150 ml). On agite environ 30 minutes et laisse sédimenter environ 30 minutes. Le surnageant est à nouveau écarté et remplacé par la même quantité de méthanol (150 ml). On agite pendant approximativement 15 minutes puis on filtre. Le gâteau est lavé par 3 fois 50 ml de méthanol, essoré puis séché pendant environ 18 h à 50°C sous pression réduite (6 kPa). On obtient 1,40 g d'HTBPM dépolymérisée brute, sel de sodium. Le rendement obtenu est de 65,8 %.

### . Saponification de l'HTBPM dépolymérisée brute, sel de sodium (étape f1 du procédé) :

1,40 g (2,18 mmoles) d'HTBPM dépolymérisée brute (sel de sodium) sont solubilisés dans 14 ml d'eau. La solution est chargée dans un ballon tricol de 100 ml. A une température voisine de 4°C, on introduit 351 µl (3,5 mmoles) de lessive de soude à 30 %. On agite pendant environ 2 h à cette température. La solution est neutralisée par addition d'acide acétique glacial (100 %). On ajoute ensuite 7 g d'acétate de sodium solide et 130 ml de méthanol. La suspension est agitée 30 minutes puis on laisse sédimenter pendant environ 1 heure. Le surnageant est écarté et remplacé par la même quantité de méthanol (80 ml). On agite encore environ 30 minutes et on laisse sédimenter approximativement 16 heures. Le surnageant est écarté et remplacé par la même quantité de méthanol (80 ml). On agite de l'ordre de 15 minutes puis on filtre sur membrane de 0,45 µm. Le gâteau est lavé par 2 fois 10 ml de méthanol, essoré puis séché pendant environ 18 h à 50°C sous pression réduite (6 kPa). On obtient 1,15 g (rendement : 89,4 %) d'HTBPM dépolymérisée brute (sel de sodium).

Le rendement obtenu est de 89,4 %.

### . Purification de l'HTBPM dépolymérisée brute -sel de sodium- (étape f2 du procédé) :

373 mg d'HTBPM dépolymérisée brute (sel de sodium) sont chargés dans un tricol de 10 ml avec 3,7 ml d'eau. La solution est portée 10 minutes à 40°C. Le pH est amené à environ 9,5 par addition de soude 1 N. La solution est filtrée sur membrane 0,45 µm puis 18 µl d'une solution aqueuse de peroxyde d'hydrogène à 30 % sont ajoutés. Le mélange est agité environ 2 h à température ambiante en maintenant le pH constant à 9,5 ± 0,1 par addition de soude 0,1 N. Le mélange réactionnel est neutralisé par HCl 0,1 N puis 430 mg de NaCl sont additionnés. Après environ 10 minutes d'agitation, la solution est filtrée sur membrane 0,45 µm. 3 ml de méthanol sont coulés à une température voisine de 4 °C. La solution est agitée 15 minutes à température ambiante. 7,7 ml de méthanol sont ensuite ajoutés. La suspension est agitée pendant environ 1 h. L'agitation est alors arrêtée et on laisse sédimenter pendant approximativement 40 minutes. Le surnageant est ensuite prélevé puis écarté (10 ml). Sur le précipité sédimenté, on ajoute 10 ml de méthanol et on agite pendant 15 minutes. On laisse re-sédimenter le précipité 30 minutes environ. Le surnageant est prélevé et écarté (10 ml). 10 ml de méthanol sont ajoutés et le précipité en suspension est ensuite filtré sur membrane 0,45 µm. Le gâteau blanc obtenu est lavé par 4 portions de 5 ml de méthanol. Le solide humide est essoré puis séché sous pression réduite (6 kPa), à une température voisine de 50°C. Après environ 18 heures de séchage, on obtient 199 mg d'HTBPM dépolymérisée pure (sel de sodium). Le rendement obtenu est de 54 %.

### Caractéristique de l'HTBPM dépolymérisée ainsi obtenue

Poids moléculaire moyen : 2000 Daltons. Indice de polydispersité : 1.1
Activité anti-Xa : 252 UI/mg

### Exemple 4 :

HTBPM obtenue par le procédé selon l'invention comprenant une étape d'estérification à 96 % et une étape de dépolymerization par le BEMP

### . Estérification de l'HTBPM (étape b) du procédé)

14,45 g (7,7 mmoles) d'HTBPM sel de benzéthonium obtenue selon la préparation 4 sont solubilisés dans 75,79 g de dichlorométhane anhydre et chargés dans un tricol de 250 ml. (La teneur en eau du milieu réactionnel est de 0,20 %). 12,4 ml (108 mmoles) de chlorure de benzyle sont ajoutés à la température de 30 °C. Le taux d'estérification est de 96 % après environ 26 h de réaction à 30°C. Après refroidissement à température ambiante, le mélange réactionnel est versé sur 180 ml d'une solution à 12 % d'acétate de sodium dans le méthanol. Le mélange est agité environ 30 minutes à température ambiante puis on laisse sédimenter approximativement 30 minutes. Le surnageant est écarté puis remplacé par le même volume de méthanol (150 ml). On agite environ 15 minutes puis on filtre. Le gâteau est lavé par 2 portions de 100 ml de méthanol, essoré puis séché pendant environ 18 h à 40°C sous pression réduite (6 kPa). On obtient 3,67 g d'HTBPM ester de benzyle, sel de sodium brut, avec un taux d'estérification de 96 %.

### . Purification de l'HTBPM ester de benzyle (estérifié à 96 %) sel de sodium (étape b) du procédé) :

Les 3,67 g d'HTBPM ester de benzyle, sel de sodium brut sont solubilisés dans 37 ml d'une solution aqueuse de NaCl à 10 % (3,7 g de NaCl dans 37 ml d'eau). La solution est coulée sur 167 ml de méthanol. La suspension est agitée environ 15 minutes puis on laisse sédimenter pendant approximativement 1 h. Le surnageant est écarté puis remplacé par le même volume de méthanol (38 ml). On agite pendant environ 5 minutes et on filtre. Le gâteau est lavé le gâteau par 2 fois 30 ml de méthanol. Le solide humide blanc est essoré, séché pendant environ 18 h à 40°C sous pression réduite (6 kPa). On obtient 2,76 g d'HTBPM ester de benzyle, sel de sodium.

Le rendement d'estérification est de 54,4 %.

### . Trans-salifcation de l'HTBPM ester de benzyle en sel de benzéthonium (étape c) du procédé) :

2,83 g (4,29 mmoles) d'HTBPM ester de benzyle, sel de sodium sont solubilisés avec 20 ml d'eau dans un erlenmeyer « A » de 100 ml. Pendant ce temps, 6,35 g (14,2 mmoles) de chlorure de benzéthonium sont chargés avec 50 ml d'eau dans un erlenmeyer « B » de 100 ml.

Le contenu de « B » est coulé dans « A ». La suspension est agitée environ 1 h à température ambiante puis on laisse sédimenter approximativement 1 h. Le surnageant est écarté et remplacé par le même volume d'eau (60 ml). On agite pendant environ 15 minutes et laisse sédimenter approximativement 1 h. Le surnageant est écarté et remplacé par le même volume d'eau (60 ml). On agite encore environ 5 minutes puis on filtre. Le gâteau est lavé par 4 fois 50 ml d'eau, essoré puis séché pendant environ 18 h à 80°C sous pression réduite (6 kPa). On obtient 7,0 g d'HTBPM ester de benzyle, sel de benzéthonium.

Le rendement observé est d'environ 100 %. La teneur en eau est de 0.23%.

### . Dépolymérisation de l'HTBPM ester de benzyl, sel de benzéthonium (étape d) du procédé) :

3,67 g (2,3 mmoles) d'HTBPM sont chargés dans un ballon tricol de 50 ml avec 27 ml de dichlorométhane sec. Le mélange est porté à 30°C. On ajoute 676 µl (2,3 mmoles) de 2-tert-butylimino-2-diethylamino-1 ,3-diméthylperhydro-1,3,2-diaza-phosphorine et on agite 24 h à 30°C.

### . Transformation du sel d'ammonium quaternaire en sel de sodium (étape e) du procédé)

Pendant ce temps, 150 ml d'une solution méthanolique d'acétate de sodium à 10 % sont préparés dans un erlenmeyer de 250 ml. Le mélange réactionnel est coulé dans la solution méthanolique en maintenant la température à environ 4°C. La suspension est agitée pendant environ 1 h à température ambiante. On laisse sédimenter pendant approximativement 1 h. Le surnageant est écarté puis remplacé par la même quantité de méthanol (100 ml). On agite 30 minutes et laisse sédimenter 30 minutes. Le surnageant est à nouveau écarté et remplacé par la même quantité de méthanol (100 ml). On agite 15 minutes et filtre. Le gâteau est lavé par 3 fois 40 ml de méthanol, essoré et séché pendant environ 18 h à 50°C sous pression réduite (6 kPa). On obtient 966 mg d'HTBPM dépolymérisée, sel de sodium.

Le rendement obtenu est de 64 %.

### . Saponification de l'HTBPM ester de benzyle, sel de sodium (étape f1 du procédé):

942 mg (1,43 mmoles) d'HTBPM dépolymérisée sel de sodium sont solubilisés dans 9,5 ml d'eau. La solution est chargée dans un ballon tricol de 100 ml. On introduit 236 µl (2,35 mmoles) de lessive de soude à 30 % à une température voisine de 4°C. On agite pendant environ 2 h à cette température. La solution est neutralisée par addition d'acide acétique glacial (100 %). On ajoute ensuite 4,5 g d'acétate de sodium solide et 85 ml de méthanol. La suspension est agitée pendant environ 30 minutes puis on laisse sédimenter pendant environ 1 heure. Le surnageant est écarté et remplacé par la même quantité de méthanol (40 ml). On agite encore pendant environ 30 minutes et on laisse sédimenter pendant environ 16 heures. Le surnageant est écarté et remplacé par la même quantité de méthanol (40 ml). On agite pendant environ 30 minutes et on filtre sur membrane de 0,45 µm. Le gâteau est lavé par 2 fois 10 ml de méthanol, essoré puis séché pendant environ 18 h à 50°C sous pression réduite (6 kPa). On obtient 776 mg d'HTBPM dépolymérisée brute (sel de sodium).

Le rendement obtenu est de 91,4 %.

### . Purification de l'HTBPM dépolymérisée brute -sel de sodium- étape f2 du procédé :

758 mg d'HTBPM dépolymérisée brute (sel de sodium) sont chargés dans un tricol de 25 ml avec 7,6 ml d'eau. La solution est portée 10 minutes à 40°C. Le pH est amené à environ 9,5 par addition de soude 0,1 N. La solution est filtrée sur membrane 0,45 µm puis 38 µl d'une solution aqueuse de peroxyde d'hydrogène à 30 % sont ajoutés. Le mélange est agité pendant environ 2 h à température ambiante en maintenant le pH constant à 9,5 ± 0,1 par addition de soude 0,1 N. Le mélange réactionnel est neutralisé par HCl 1N et 880 mg de NaCl sont additionnés. Après environ 10 minutes d'agitation, la solution est filtrée sur membrane 0,45 µm. 6,2 ml de méthanol sont coulés à une température voisine de 4 °C. La solution est agitée approximativement 15 minutes à température ambiante. On ajoute 16 ml de méthanol et la suspension est agitée pendant environ 1 h. L'agitation est alors arrêtée et la suspension est filtrée. Le gâteau est lavé par 2 portions de 15 ml de méthanol. Le solide humide est essoré puis séché sous pression réduite (6 kPa), à une température voisine de 50°C. Après approximativement 18 heures de séchage, on obtient 490 mg d'HTBPM dépolymérisée pure (sel de sodium). Le rendement obtenu est de 65 %.

### Caractéristique de l'HTBPM dépolymérisée ainsi obtenue

Poids moléculaire moyen : 2000 Daltons
Indice de polydispersité : 1.1
Activité anti-Xa : 205 UI/mg

### Exemple 5 :

HTBPM obtenue par le procédé selon l'invention comprenant une étape d'estérification à 96 % et une étape de dépolymerization par le tert-Butylimino-tris(dimethyamino) phosphorane

### . Dépolymérisation de l'HTBPM ester de benzyle, sel de benzéthonium (étape d) du procède):

3,67 g (2,3 mmoles) d'HTBPM ester de benzyle, sel de benzéthonium obtenue selon l'exemple 4 (estérifié à 96 %) avec une teneur en eau de 0,23 %, sont chargés dans un tricol de 50 ml avec 30 ml de dichlorométhane sec. Le mélange est porté à 30°C. On ajoute 595 µl (2,3 mmoles) de tert-Butylimino-tris(dimethylamino) phosphorane et on agite 24 h à 30°C.

### . Transformation du sel d'ammonium quaternaire en sel de sodium (étape e) du procédé)

Pendant ce temps, 160 ml d'une solution méthanolique d'acétate de sodium à 10 % sont préparés dans un erlenmeyer de 250 ml. Le mélange réactionnel est coulé dans la solution méthanolique en maintenant la température à environ 4°C. La suspension est agitée approximativement 1 h à température ambiante. On laisse sédimenter pendant environ 1 h. Le surnageant est écarté puis remplacé par la même quantité de méthanol (120 ml). On agite environ 30 minutes et laisse sédimenter 30 minutes. Le surnageant est à nouveau écarté et remplacé par la même quantité de méthanol (125 ml). On agite pendant environ 15 minutes et on filtre. Le gâteau est lavé par 3 fois 40 ml de méthanol, essoré et séché environ 18 h à une température voisine de 50°C sous pression réduite (6 kPa). On obtient 982 mg d'HTBPM dépolymérisée brute, sel de sodium. Le rendement obtenu est de 65 %.

### . Saponification de l'HTBPM dépolymérisée brute, sel de sodium (étape f1 du procédé) :

980 mg (1,49 mmoles) d'HTBPM dépolymérisée brute, sel de sodium, sont solubilisés dans 10 ml d'eau. La solution est chargée dans un ballon tricol de 100 ml. On introduit 246 µl (2,45 mmoles) de lessive de soude à 30 % à une température voisine de 4°C. On agite pendant environ 2 h à cette température. La solution est neutralisée par addition d'acide acétique glacial (100 %). On ajoute ensuite 4,9 g d'acétate de sodium solide et 95 ml de méthanol. La suspension est agitée 30 minutes puis on laisse sédimenter pendant environ 1 heure. Le surnageant est écarté puis remplacé par la même quantité de méthanol (60 ml). On agite encore environ 30 minutes puis on laisse sédimenter environ 16 heures. Le surnageant est écarté puis remplacé par la même quantité de méthanol (60 ml). On agite pendant environ 30 minutes et on filtre sur membrane de 0,45 µm. Le gâteau est lavé par 2 fois 10 ml de méthanol, essoré puis séché approximativement 18 h à 50°C sous pression réduite (6 kPa). On obtient 809 mg d'HTBPM dépolymérisée brute, sel de sodium.

Le rendement de la réaction est de 91,6 %.

### . Purification de l'HTBPM dépolymérisée brute -sel de sodium- étape f2 du procédé :

792 mg d'HTBPM dépolymérisée brute (sel de sodium) sont chargés dans un tricol de 25 ml avec 8 ml d'eau. La solution est portée 10 minutes à 40 °C. Le pH est amené à environ 9,5 par addition de soude 0,1 N. La solution est filtrée sur membrane 0,45 µm, puis 39,6 µl d'une solution aqueuse de peroxyde d'hydrogène à 30 % sont ajoutés. Le mélange est agité 2 h à température ambiante en maintenant le pH constant à 9,5 ± 0,1 par addition de soude 0,1 N. Le mélange réactionnel est neutralisé par HCl 1N et 1,04 g de NaCl sont additionnés. Après environ 10 minutes d'agitation, la solution est filtrée sur membrane 0,45 µm. 7,3 ml de méthanol sont coulés à une température voisine de 4°C. La solution est agitée pendant 15 minutes à température ambiante. On ajoute 18,8 ml de méthanol et la suspension est agitée pendant environ 1 h. L'agitation est alors arrêtée puis on filtre. Le gâteau est lavé par 3 portions de 15 ml de méthanol. Le solide humide est essoré puis séché sous pression réduite (6 kPa), à une température voisine de 50°C. Après 18 heures de séchage, on obtient 538 mg d'HTBPM dépolymérisée pure (sel de sodium). Le rendement obtenu est de 67,9 %.

### Caractéristique de l'HTBPM dépolymérisée ainsi obtenue

Poids moléculaire moyen : 2100 Daltons
Indice de polydispersité : 1.1
Activité anti-Xa : 209 UI/mg

### Exemple 6 :

HTBPM obtenue par le procédé selon l'invention comprenant une étape supplémentaire de séparation par chromatographie en éliminant les fractions disaccharides et tetrasaccharides

Le mélange d'oligosaccharides décrit à l'exemple 1 (286 mg) est solubilisé dans 20 ml de phase mobile (solution aqueuse de bicarbonate de sodium à 0.2 moles /l). Les conditions chromatographiques sont les suivantes :
- Phase mobile : solution de bicarbonate de sodium à 0.2 moles/ 1
- Phase stationnaire : Gel biogel P6
- Colonne : Longueur 1 m, Diamètre 5 cm.
- Longueur d'onde de détection : 240 nm.

Les fractions supérieures ou égales à un hexasaccharide sont collectées et rassemblées. Elles sont neutralisées par de l'acide acétique puis concentrées jusqu'à l'obtention d'une solution à 200 g/ 1 d'acétate de sodium. A la solution obtenue et sous agitation, on ajoute 5 volumes de méthanol. La suspension est agitée pendant environ 18h, puis fitrée sur membrane 0,45 µm. Le gâteau est séché pendant environ 6 h à une température voisine de 40°C sous pression réduite (6kPa). Le produit obtenu est re-précipité puis dissous dans un minimum d'eau et désalé sur colonne Sephadex G10. Après concentration des fractions désalées puis lyophilisation, on obtient 109 mg de produit. Le rendement est de 38 %.

Les caractéristiques du mélange d'oligosaccharides ainsi obtenu sont les suivantes :
Activité anti-Xa : 403 UI/mg

Le pourcentage des oligosaccharides est le suivant :

| Mw | Poly | Di | Tetra | Hexa | Octa | Deca | > Deca |
|---|---|---|---|---|---|---|---|
| (Da) | dispersité | % | % | % | % | % | % |
| 2150 | 1.0 | 0 | 0 | 53.83 | 32.58 | 10.5 | 3.5 |

Activité pharmacologique des composés selon l'invention :

| Exemples | Poids moléculaire moyen | Activité Anti Xa | Activité Anti IIa |
|---|---|---|---|
| | | UI/mg | |
| 1 | 1950 | 283 | < 0,2 |
| 2 | 2100 | 191 | 0 |
| 3 | 2000 | 252 | 0 |
| 4 | 2000 | 205 | 0 |
| 5 | 2100 | 209 | 0 |
| 6 | 2150 | 403 | 0 |

Pourcentage en hexasaccharide Δ IIa-IIs-Is des composés selon l'invention :

| Exemples | Pourcentage de fraction hexasaccharide | Pourcentage d'hexasaccharide Δ IIa-IIs-Is dans fraction hexasaccharide |
|---|---|---|
| 1 | 31 % | 46% |
| 2 | 30 % | 26% |
| 3 | 33% | 33,5% |
| 4 | 32% | 30,8% |
| 5 | 31,5% | 28,7% |
| 6 | 53,8% | 46% |

## Revendications

1. Mélanges d'oligosaccharides possédant la structure générale des polysaccharides constitutifs de l'héparine et présentant les caractéristiques suivantes:
- un poids moléculaire moyen de 1800 à 2400 Daltons, déterminé par chromatographie liquide haute pression en utilisant deux colonnes en série,
- une activité anti-Xa comprise entre 190 UI/mg et 450 UI/mg, mesurée par rapport à une héparine de très bas poids moléculaire standard qui titre de 140 à 180 U/mg,
- une activité anti-IIa inférieure à 0,2 UI/mg, mesurée par la méthode amidolytique sur un substrat chromogénique selon la méthode décrite dans la monographie des héparines de basse masse moléculaire de la pharmacopée européenne,
- étant entendu que les oligosaccharides constitutifs des mélanges
- contiennent de 2 à 16 motifs saccharidiques,
- ont un motif acide uronique-4,5 insaturé 2-O-sulfate à l'une de leurs extrémités,
- et contiennent l'hexasaccharide de formule suivante : 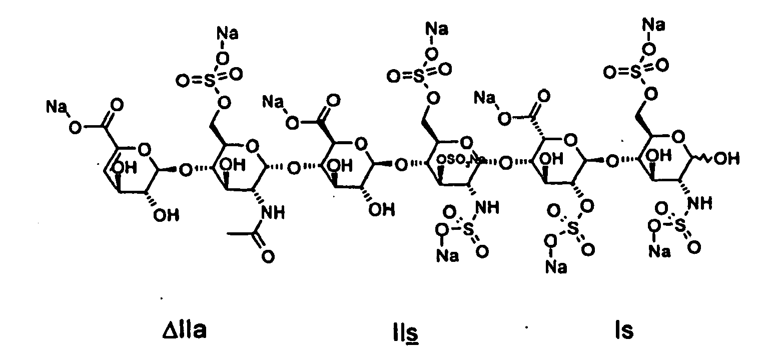 sous forme d'un sel de métal alcalin ou alcalinoterreux.

2. Mélanges d'oligosaccharides selon la revendication 1 **caractérisés en ce que** les sels de métal alcalin ou alcalinoterreux sont les sels de sodium, potassium, calcium et magnésium.

3. Mélanges d'oligosaccharides selon la revendication 1 ou 2 **caractérisés en ce qu'**ils contiennent de 20 à 100 %, et en particulier de 30 à 60 %, de fraction hexasaccharide.

4. Mélanges d'oligosaccharides selon la revendication 3 **caractérisés en ce que** la fraction hexasaccharide contient de 20 à 70 %, et en particulier de 25 à 50 %, de l'hexasaccharide ΔIIa-IIs-Is tel que défini dans la revendication 1.

5. Mélanges d'oligosaccharides selon l'une quelconque des revendications 1 à 4 **caracterisés en ce qu**'ils ont un poids moléculaire moyen compris entre 1900 et 2200 et en particulier de 1950 et 2150 Daltons.

6. Mélanges d'oligosaccharides selon l'une quelconque des revendications 1 à 5 **caractérisés en ce qu'**ils ont une activité anti-Xa comprise entre 190 UI/mg et 410 UI/mg, et en particulier entre 200 et 300 UI/mg, tout en présentant une activité anti-IIa inférieure à 0,2 UI/mg.

7. Mélanges d'oligosaccharides selon l'une quelconque des revendications 1 à 6 **caractérisé en ce qu'**ils présentent les caractéristiques suivantes :
- un poids moléculaire moyen compris entre 1950 à 2150 Daltons,
- une activité anti-Xa comprise entre 190 UI/mg et 410 UI/mg et une activité anti-IIa inférieure à 0,2 UI/mg,
- ils renferment de 30 à 60 % de fraction hexasaccharide contenant de 25 à 55 % de fraction ΔIIa-IIs-Is.

8. Procédé de préparation des mélanges d'oligosaccharides selon l'une quelconque des revendications 1 à 7 **caractérisé en ce qu'**une héparine de très bas poids moléculaire ayant :
une activité anti-Xa supérieure à 140 UI /mg, mesurée par rapport à une héparine de très bas poids moléculaire standard qui titre de 140 à 180 U/mg,
une activité anti-IIa inférieure à 5 UI/mg, mesurée par la méthode amidolytique sur un substrat chromogénique selon la méthode décrite dans la monographie des héparines de basse masse moléculaire de la pharmacopée européenne, et
une masse moléculaire moyenne comprise entre 2000 et 3000 Daltons, déterminée par chromatographie liquide haute pression en utilisant deux colonnes en série,
subit les réactions chimiques suivantes :
a) Transalification par action de chlorure de benzéthonium pour obtenir l'héparinate de benzétonium,
b) Estérification de l'héparinate de benzéthonium obtenu par action de chlorure de benzyle, et traitement par une solution alcoolique d'acétate de sodium pour obtenir le sel de sodium de l'ester benzylique de l'héparine de très bas poids moléculaire
c) Transalification de l'ester benzylique obtenu et obtention du sel d'ammonium quaternaire,
d) Dépolymérisation au moyen d'une base organique forte de pka de préférence supérieur à 20 afin d'obtenir une héparine de très bas poids moléculaire dépolymérisée,
e) Transformation du sel d'ammonium quaternaire de l'héparine de très bas poids moléculaire dépolymérisée en sel de sodium
f) Saponification des esters résiduels et éventuellement purification.

9. Procédé de préparation selon la revendication 8 **caractérisé en ce que** le rapport en mole base forte/ester utilisé lors de l'étape de polymérisation d) est compris entre 0,2 et 5 et, de préférence, entre 0,6 et 2.

10. Procédé de préparation selon la revendication 8 ou 9 **caractérisé en ce** l'étape de dépolymérisation d) s'effectue avec comme base un dérivé du phosphazène.

11. Procédé de préparation selon l'une quelconque des revendications 8 à 10 **caractérisé en ce** l'étape de dépolymérisation d) s'effectue avec des teneurs en eau inférieures à 0,3 % lorsque l'on opère avec 1 équivalent molaire de base phosphazène par rapport à l'ester benzylique de l'héparine de très bas poids moléculaire, sel de benzéthonium.

12. Procédé de préparation selon l'une quelconque des revendications 8 à 11 **caractérisé en ce que** les bases de la famille des phosphazènes utilisées lors de l'étape de dépolymérisation d) sont, de préférence, celles de formule : 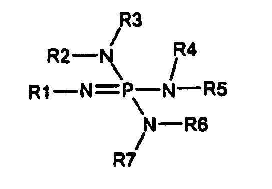 dans laquelle les radicaux R₁ à R₇, identiques ou différents, représentent des radicaux alkyles linéaires, ramifiés ou cycliques contenant de 1 à 6 atomes de carbone, R₃ et R₄ pouvant, le cas échéant, former avec le groupement N-P-N- qui les porte un hétérocycle à 6 chaînons.

13. Procédé de préparation selon l'une quelconque des revendications 8 à 12 **caractérisé en ce que** la base de la famille des phosphazènes utilisée lors de l'étape de dépolymérisation est le 2-tert-butylimino-2-diethylamino-1,3-diméthylperhydro-1,3,2-diaza-phosphorine.

14. Procédé de préparation selon la revendication 8 **caractérisé en ce que** le taux d'estérification du sel d'ammonium quaternaire de l'ester benzylique de l'héparine lors de l'étape b) est compris entre 40 et 100 % et, de préférence, entre 70 et 90 %.

15. Procédé de préparation selon la revendication 8 **caractérisé en ce que** la transformation du sel d'ammonium quaternaire de l'ester benzylique de l'héparine de très bas poids moléculaire telle que préparée selon l'étape b) du procédé selon la revendication 8 en sel de sodium est effectuée par traitement du milieu réactionnel avec une solution alcoolique d'acétate de sodium à 10 % d'acétate de sodium dans le méthanol (poids/volume), à une température comprise entre 15 et 25°C.

16. Procédé de préparation selon la revendication 15 **caractérisé en ce que** l'équivalent en poids d'acétate de sodium ajouté lors de l'étape d'estérification b) est 3 fois supérieur à la masse de sel d'ammonium quaternaire de l'ester benzylique de l'héparine engagé dans la réaction de dépolymérisation.

17. Procédé de préparation selon la revendication 8 des mélanges d'oligosaccharides **caractérisé en ce que** le sel d'ammonium quaternaire de l'ester benzylique de l'héparine de très bas poids moléculaire obtenu lors de l'étape c) est de préférence le sel de benzéthonium, de cétylpyridinium ou de cétyltriméthyl ammonium.

18. Procédé de préparation selon la revendication 8 **caractérisé en ce que** la saponification (selon l'étape f) s'effectue au moyen d'un hydroxyde de métal alcalin tel que la soude, la potasse, l'hydroxyde de lithium, en milieu aqueux, à une température comprise entre 0 et 20°C et de préférence 0 et 10°C.

19. Procédé de préparation selon la revendication 18 **caractérisé en ce qu'**on utilise de 1 à 5 équivalents molaires d'hydroxyde de métal alcalin et plus particulièrement de 1 à 2 équivalents molaires d'hydroxyde de métal alcalin.

20. Procédé, selon l'une quelconque des revendications 8 à 19, de préparation des mélanges d'oligosaccharides tels que décrits à l'une quelconque des revendications 1 à 5 possédant une sélectivité vis-à-vis du facteur Xa augmentée **caractérisé en ce qu'**en outre on élimine les fractions disaccharides et tétrasaccharides par chromatographie notamment sur des colonnes remplies de gel de type polyacrylamide agarose.

21. Procédé de préparation selon l'une quelconque des revendications 8 à 20 des mélanges d'oligosaccharides selon l'une quelconque des revendications 1 à 7 **caractérisé en ce qu'**on utilise à titre de produit de départ une héparine de bas poids moléculaire, ou une héparine de très bas poids moléculaire (1500 à 3000 Da) ayant une activité anti-Xa comprise entre 100 et 140 UI/mg au lieu de l'héparine de très bas poids moléculaire telle que définie à la revendication 8.

22. Procédé de préparation selon l'une quelconque des revendications 8 à 20 des mélanges d'oligosaccharides selon l'une quelconque des revendications 1 à 7 **caractérisé en ce qu'**on utilise à titre de produit de départ une héparine de très bas poids moléculaire (1500 à 4000 Da) ayant une activité anti-Xa comprise entre 100 et 140 UI/mg au lieu de l'héparine de très bas poids moléculaire telle que définie à la revendication 8.

23. Procédé de préparation selon la revendication 21 **caractérisé en ce que** l'héparine de bas poids moléculaire est choisie parmi l'Enoxaparine, la Fraxiparine, la Fragmine, l'Innohep (ou Logiparin), le Normiflo, l'Embollex (ou Sandoparin), le Fluxum (ou Minidalton), la Clivarine et l'Hibor.

24. Médicament, **caractérisé en ce qu'**il comprend un mélange d'oligosaccharide selon l'une quelconque des revendications 1 à 7.

25. Médicament selon la revendication 24 pour le traitement ou la prévention des thromboses veineuses et artérielles, la thrombose veineuse profonde, l'embolie pulmonaire, l'angor instable, l'infarctus du myocarde, l'ischémie cardiaque, les maladies occlusives des artères périphériques et la fibrillation auriculaire, de la prolifération des cellules musculaires lisses l'athérosclérose et l'artériosclérose, du cancer par la modulation de l'angiogénèse et des facteurs de croissance, ainsi que des désordres diabétiques tel que les rétinopathies et néphropathies diabétiques.

26. Composition pharmaceutique renfermant au moins un médicament tel que défini à la revendication 24 et un ou plusieurs excipients ou véhicules ou additifs pharmaceutiquement inertes.

27. Composition pharmaceutique selon la revendication 26 **caractérisée en ce qu'**elle consiste en une solution injectable par voie sous-cutanée ou intraveineuse.

28. Composition pharmaceutique selon la revendication 26 **caractérisée en ce qu'**elle consiste en une formulation par inhalation destinée à la voie pulmonaire.

29. Composition pharmaceutique selon la revendication 26 **caractérisée en ce qu'**elle consiste en une formulation destinée à la voie orale.

## Claims

1. Oligosaccharide mixtures having the general structure of the constituent polysaccharides of heparin and having the following characteristics:
- an average molecular weight of 1800 to 2400 daltons, determined by high-pressure liquid chromatography using two columns in series,
- an anti-Xa activity of between 190 IU/mg and 450 IU/mg, measured relative to a standard very low molecular weight heparin with a titre of 140 to 180 U/mg,
- an anti-IIa activity of less than 0.2 IU/mg, measured by the amidolytic method on a chromogenic substrate according to the method described in the monograph on low molecular mass heparins of the European pharmacopoeia,
- it being understood that the constituent oligosaccharides of the mixtures
- contain from 2 to 16 saccharide units,
- have a 4,5-unsaturated uronic acid 2-O-sulfate unit at one of their ends,
- and contain the hexasaccharide of the following formula: 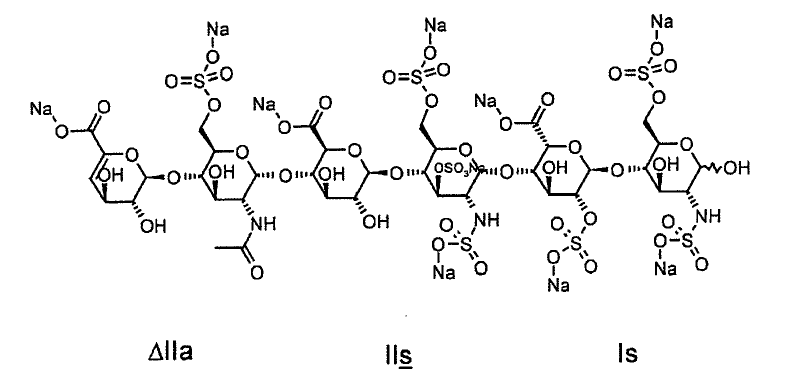
in the form of an alkali metal or alkaline-earth metal salt.

2. Oligosaccharide mixtures according to Claim 1, **characterized in that** the alkali metal or alkaline-earth metal salts are the sodium, potassium, calcium and magnesium salts.

3. Oligosaccharide mixtures according to Claim 1 or 2, **characterized in that** they contain from 20% to 100% and in particular from 30% to 60% of hexasaccharide fraction.

4. Oligosaccharide mixtures according to Claim 3, **characterized in that** the hexasaccharide fraction contains from 20% to 70% and in particular from 25% to 50% of the hexasaccharide ΔIIa-IIs-Is as defined in Claim 1.

5. Oligosaccharide mixtures according to any one of Claims 1 to 4, **characterized in that** they have an average molecular weight of between 1900 and 2200 and in particular from 1950 to 2150 daltons.

6. Oligosaccharide mixtures according to any one of Claims 1 to 5, **characterized in that** they have anti-Xa activity of between 190 IU/mg and 410 IU/mg and in particular between 200 and 300 IU/mg, while at the same time having an anti-IIa activity of less than 0.2 IU/mg.

7. Oligosaccharide mixtures according to any one of Claims 1 to 6, **characterized in that** they have the following characteristics:
- an average molecular weight of between 1950 and 2150 daltons,
- anti-Xa activity of between 190 IU/mg and 410 IU/mg and an anti-IIa activity of less than 0.2 IU/mg,
- they contain from 30% to 60% of hexasaccharide fraction, which contains from 25% to 55% of ΔIIa-IIs-Is fraction.

8. Process for preparing the oligosaccharide mixtures according to any one of Claims 1 to 7, **characterized in that** a very low molecular weight heparin having:
- an anti-Xa activity of greater than 140 IU/mg, measured relative to a standard very low molecular weight heparin with a titre of 140 to 180 U/mg,
- an anti-IIa activity of less than 5 IU/mg, measured by the amidolytic method on a chromogenic substrate according to the method described in the monograph on low molecular mass heparins of the European pharmacopoeia, and
- an average molecular mass of 2000 to 3000 daltons, determined by high-pressure liquid chromatography using two columns in series,
is subjected to the following chemical reactions:
a) transsalification by the action of benzethonium chloride to obtain benzethonium heparinate,
b) esterification of the benzethonium heparinate obtained by the action of benzyl chloride, and treatment with an alcoholic sodium acetate solution to obtain the sodium salt of the benzyl ester of the very low molecular weight heparin,
c) transsalification of the benzyl ester obtained and production of the quaternary ammonium salt,
d) depolymerization by means of a strong organic base with a pKa value preferably of greater than 20, so as to obtain a depolymerized very low molecular weight heparin,
e) conversion of the quaternary ammonium salt of the depolymerized very low molecular weight heparin into the sodium salt,
f) saponification of the residual esters and optional purification.

9. Preparation process according to Claim 8, **characterized in that** the strong base/ester mole ratio used during the polymerization step d) is between 0.2 and 5 and preferably between 0.6 and 2.

10. Preparation process according to Claim 8 or 9, **characterized in that** the depolymerization step d) is performed using a phosphazene derivative as base.

11. Preparation process according to any one of Claims 8 to 10, **characterized in that** the depolymerization step d) is performed with water contents of less than 0.3% when the process is performed with 1 molar equivalent of phosphazene base relative to the benzyl ester of the very low molecular weight heparin, benzethonium salt.

12. Preparation process according to any one of Claims 8 to 11, **characterized in that** the bases of the phosphazene family used during the depolymerization step d) are preferably those of formula: 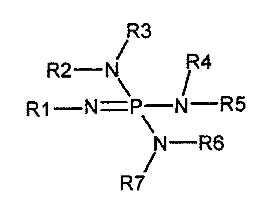 in which the radicals R₁ to R₇, which are identical or different, represent linear, branched or cyclic alkyl radicals containing from 1 to 6 carbon atoms, it being possible for R₃ and R₄, where appropriate, to form, with the -N-P-N group which carries them, a 6-membered heterocycle.

13. Preparation process according to any one of Claims 8 to 12, **characterized in that** the base of the family of phosphazenes used in the depolymerization step is 2-tert-butylimino-2-diethylamino-1,3-dimethylperhydro-1,3,2-diazaphosphorine.

14. Preparation process according to Claim 8, **characterized in that** the degree of esterification of the quaternary ammonium salt of the benzyl ester of heparin during step b) is between 40% and 100% and preferably between 70% and 90%.

15. Preparation process according to Claim 8, **characterized in that** the conversion of the quaternary ammonium salt of the benzyl ester of the very low molecular weight heparin as prepared according to step b) of the process according to Claim 8, into the sodium salt, is performed by treating the reaction medium with an alcoholic sodium acetate solution 10% sodium acetate in methanol (weight/volume), at a temperature of between 15 and 25°C.

16. Preparation process according to Claim 15, **characterized in that** the weight equivalent of sodium acetate added during the esterification step b) is 3 times as great as the mass of quaternary ammonium salt of the benzyl ester of heparin used in the depolymerization reaction.

17. Process according to Claim 8 for preparing the oligosaccharide mixtures, **characterized in that** the quaternary ammonium salt of the benzyl ester of the very low molecular weight heparin obtained during step c) is preferably the benzethonium, cetylpyridinium or cetyltrimethylammonium salt.

18. Preparation process according to Claim 8, **characterized in that** the saponification (according to step f) is performed using an alkali metal hydroxide such as sodium hydroxide, potassium hydroxide or lithium hydroxide, in aqueous medium, at a temperature of between 0 and 20°C and preferably between 0 and 10°C.

19. Preparation process according to Claim 18, **characterized in that** from 1 to 5 molar equivalents of alkali metal hydroxide and more particularly from 1 to 2 molar equivalents of alkali metal hydroxide are used.

20. Process, according to any one of Claims 8 to 19, for preparing the oligosaccharide mixtures as described in any one of Claims 1 to 5 having increased selectivity toward factor Xa, **characterized in that** the disaccharide and tetrasaccharide fractions are also removed by chromatography, especially on columns filled with gel of polyacrylamide agarose type.

21. Process according to any one of Claims 8 to 20 for preparing the oligosaccharide mixtures according to any one of Claims 1 to 7, **characterized in that** a low molecular weight heparin is used as starting material, or a very low molecular weight heparin (1500 to 3000 Da) with an anti-Xa activity of between 100 and 140 IU/mg instead of the very low molecular weight heparin as defined in Claim 8.

22. Process according to any one of Claims 8 to 20 for preparing the oligosaccharide mixtures according to any one of claims 1 to 7, **characterized in that** a very low molecular weight heparin (1500 to 4000 Da) with an anti-Xa activity of between 100 and 140 IU/mg is used as starting material instead of the very low molecular weight heparin as defined in Claim 8.

23. Preparation process according to Claim 21, **characterized in that** the low molecular weight heparin is chosen from Enoxaparin, Fraxiparin, Fragmin, Innohep (or Logiparin), Normiflo, Embollex (or Sandoparin), Fluxum (or Minidalton), Clivarin and Hibor.

24. Medicinal product, **characterized in that** it comprises a oligosaccharide mixture according to any one of Claims 1 to 7.

25. Medicinal product according to Claim 24, for treating or preventing venous and arterial thrombosis, deep vein thrombosis, pulmonary embolism, unstable angina, myocardial infarction, cardiac ischemia, occlusive diseases of the peripheral arteries and atrial fibrillation, smooth muscle cell proliferation, atherosclerosis and arteriosclerosis, cancer by modulating angiogenesis and growth factors, and also diabetic disorders such as diabetic retinopathy and diabetic nephropathy.

26. Pharmaceutical composition containing at least one medicinal product as defined in Claim 24 and one or more pharmaceutically inert excipients or vehicles or additives.

27. Pharmaceutical composition according to Claim 26, **characterized in that** it consists of a solution for subcutaneous or intravenous injection.

28. Pharmaceutical composition according to Claim 26, **characterized in that** it consists of a pulmonary formulation for inhalation.

29. Pharmaceutical composition according to Claim 26, **characterized in that** it consists of an oral formulation.

## Patentansprüche

1. Gemische von Oligosacchariden mit der allgemeinen Struktur der Polysaccharide, die Heparin aufbauen, und mit den folgenden Eigenschaften:
- einer mittleren Molekülmasse von 1800 bis 2400 Dalton, bestimmt mittels Hochdruckflüssigkeitschromatographie unter Verwendung zweier in Reihe verbundener Säulen,
- einer Anti-Xa-Aktivität zwischen 190 IU/mg und 450 IU/mg, gemessen im Vergleich zu einem sehr niedermolekularen Heparin-Standard, der 140 bis 180 U/mg aufweist,
- einer Anti-IIa-Aktivität unter 0,2 IU/mg, gemessen durch das amidolytische Verfahren an einem chromogenen Substrat gemäß dem Verfahren, das in der Monographie zu niedermolekularen Heparinen der europäischen Pharmakopöe beschrieben ist,
wobei selbstverständlich die Oligosaccharide, die die Gemische ausmachen:
- 2 bis 16 Saccharidmotive enthalten,
- ein 4,5-ungesättigte-Uronsäure-2-O-sulfat an einem ihrer Enden aufweisen,
- und das Hexasaccharid der folgenden Formel: 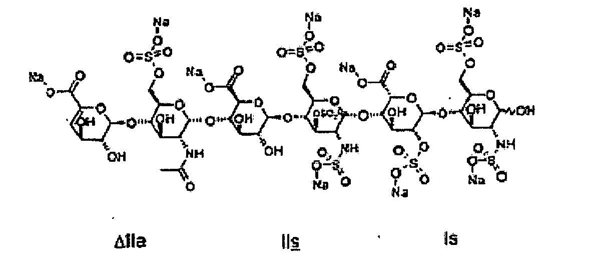
in Form eines Alkali- oder Erdalkalimetallsalzes enthalten.

2. Oligosaccharidgemische nach Anspruch 1, **dadurch gekennzeichnet, dass** es sich bei den Alkali- oder Erdalkalimetallsalzen um die Natrium-, Calcium- und Magnesiumsalze handelt.

3. Oligosaccharidgemische nach Anspruch 1 oder 2, **dadurch gekennzeichnet, dass** sie 20 bis 100% und insbesondere 30 bis 60% der Hexasaccharidfraktion enthalten.

4. Oligosaccharidgemische nach Anspruch 3, **dadurch gekennzeichnet, dass** die Hexasaccharidfraktion 20 bis 70% und insbesondere 25 bis 50% des Hexasaccharids ΔIIa-IIs-Is, wie im Anspruch 1 definiert, enthält.

5. Oligosaccharidgemische nach einem der Ansprüche 1 bis 4, **dadurch gekennzeichnet, dass** sie eine mittlere Molekülmasse zwischen 1900 und 2200 und insbesondere von 1950 bis 2150 Dalton aufweisen.

6. Oligosaccharidgemische nach einem der Ansprüche 1 bis 5, **dadurch gekennzeichnet, dass** sie eine Anti-Xa-Aktivität zwischen 190 IU/mg und 410 IU/mg und insbesondere zwischen 200 und 300 IU/mg aufweisen, wobei sie gleichzeitig eine Anti-IIa-Aktivität unter 0,2 IU/mg aufweisen.

7. Oligosaccharidgemische nach einem der Ansprüche 1 bis 6, **dadurch gekennzeichnet, dass** sie die folgenden Eigenschaften aufweisen:
- eine mittlere Molekülmasse zwischen 1950 bis 2150 Dalton,
- eine Anti-Xa-Aktivität zwischen 190 IU/mg und 410 IU/mg und eine Anti-IIa-Aktivität unter 0,2 IU/mg,
- sie enthalten 30 bis 60% der Hexasaccharidfraktion, die 25 bis 55% der ΔIIa-IIs-Is-Fraktion enthält.

8. Verfahren zur Herstellung von Oligosaccharidgemischen nach einem der Ansprüche 1 bis 7, **dadurch gekennzeichnet, dass** ein sehr niedermolekulares Heparin mit:
- einer Anti-Xa-Aktivität über 140 IU/mg, gemessen im Vergleich zu einem sehr niedermolekularen Heparin-Standard, der 140 bis 180 U/mg aufweist,
- einer Anti-IIa-Aktivität unter 5 IU/mg, gemessen durch das amidolytische Verfahren an einem chromogenen Substrat gemäß dem Verfahren, das in der Monographie zu niedermolekularen Heparinen der europäischen Pharmakopöe beschrieben ist, und
- einer mittleren Molekülmasse zwischen 2000 und 3000 Dalton, bestimmt mittels Hochdruckflüssigkeitschromatographie unter Verwendung zweier in Reihe verbundener Säulen,
den folgenden chemischen Reaktionen unterworfen wird:
a) Umsalzung durch Einwirken von Benzethoniumchlorid, um Benzethoniumheparinat zu erhalten,
b) Veresterung des erhaltenen Benzethoniumheparinats durch Einwirkung von Benzylchlorid und Behandlung mit einer alkoholischen Natriumacetatlösung, um das Natriumsalz des Benzylesters von sehr niedermolekularem Heparin zu erhalten,
c) Umsalzung des erhaltenen Benzylesters und Gewinnen des quaternären Ammoniumsalzes,
d) Depolymerisation mithilfe einer starken organischen Base mit einer pKa, die vorzugsweise größer als 20 ist, um ein depolymerisiertes sehr niedermolekulares Heparin zu erhalten,
e) Umwandeln des quaternären Ammoniumsalzes des depolymerisierten sehr niedermolekularen Heparins in das Natriumsalz,
f) Verseifung der Esterreste und gegebenenfalls Reinigung.

9. Herstellungsverfahren nach Anspruch 8, **dadurch gekennzeichnet, dass** das Molverhältnis von starker Base/Ester, das während des Polymerisationsschritts d) eingesetzt wird, zwischen 0,2 und 5 und vorzugsweise zwischen 0,6 und 2 beträgt.

10. Herstellungsverfahren nach Anspruch 8 oder 9, **dadurch gekennzeichnet, dass** der Depolymerisationsschritt d) mit einem Phosphazen-Derivat als Base erfolgt.

11. Herstellungsverfahren nach einem der Ansprüche 8 bis 10, **dadurch gekennzeichnet, dass** der Depolymerisationsschritt d) mit Wassergehalten von weniger als 0,3% erfolgt, wenn man mit 1 Moläquivalent an Phosphazenbase, bezogen auf den Benzylester von sehr niedermolekularem Heparin, Benzethoniumsalz, arbeitet.

12. Herstellungsverfahren nach einem der Ansprüche 8 bis 11, **dadurch gekennzeichnet, dass** es sich bei den im Depolymerisationsschritt d) eingesetzten Basen aus der Familie der Phosphazene vorzugsweise um diejenigen der folgenden Formel handelt: 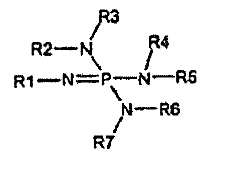 worin die Reste R₁ bis R₇, die gleich oder verschieden sind, für gerade, verzweigte oder zyklische Alkylreste mit 1 bis 6 Kohlenstoffatomen stehen, wobei R₃ und R₄ gegebenenfalls die Gruppe -N-P-N- bilden können, die einen Heterozyklus mit 6 Ringatomen trägt.

13. Herstellungsverfahren nach einem der Ansprüche 8 bis 12, **dadurch gekennzeichnet, dass** es sich bei der im Depolymerisationsschritt eingesetzten Base aus der Familie der Phosphazene um 2-tert-Butylimino-2-diethylamino-1,3-dimethylperhydro-1,3,2-diazaphosphorin handelt.

14. Herstellungsverfahren nach Anspruch 8, **dadurch gekennzeichnet, dass** der Grad der Veresterung des quaternären Ammoniumsalzes des Benzylesters von Heparin im Schritt b) zwischen 40 und 100% und vorzugsweise zwischen 70 und 90% beträgt.

15. Herstellungsverfahren nach Anspruch 8, **dadurch gekennzeichnet, dass** die Umwandlung des quaternären Ammoniumsalzes des Benzylesters von sehr niedermolekularem Heparin, wie er im Schritt b) des Verfahrens nach Anspruch 8 hergestellt wird, in das Natriumsalz durch Behandlung des Reaktionsmediums mit einer alkoholischen Natriumacetatlösung mit 10% Natriumacetat in Methanol (Gewicht/Volumen) bei einer Temperatur zwischen 15 und 25°C durchgeführt wird.

16. Herstellungsverfahren nach Anspruch 15, **dadurch gekennzeichnet, dass** das Gewichtsäquivalent an Natriumacetat, das im Veresterungsschritt b) hinzugefügt wird, 3 Mal größer ist als die Masse an quaternärem Ammoniumsalz des Benzylesters von Heparin, das in der Depolymerisationsreaktion eingesetzt wird.

17. Herstellungsverfahren nach Anspruch 8 für Oligosaccharidgemische, **dadurch gekennzeichnet, dass** das quaternäre Ammoniumsalz des Benzylesters von sehr niedermolekularem Heparin, das im Schritt c) erhalten wird, vorzugsweise das Benzethonium-, Cetylpyridinium-oder Cetyltrimethylammoniumsalz ist.

18. Herstellungsverfahren nach Anspruch 8, **dadurch gekennzeichnet, dass** die Verseifung (gemäß Schritt f) mithilfe eines Alkalimetallhydroxids, wie Natronlauge, Kalilauge, Lithiumhydroxid, in wässrigem Medium bei einer Temperatur zwischen 0 und 20°C und vorzugsweise 0 und 10°C durchgeführt wird.

19. Herstellungsverfahren nach Anspruch 18, **dadurch gekennzeichnet, dass** man 1 bis 5 Moläquivalente Alkalimetallhydroxid und insbesondere 1 bis 2 Moläquivalente Alkalimetallhydroxid verwendet.

20. Verfahren nach einem der Ansprüche 8 bis 19 zur Herstellung von Oligosaccharidgemischen nach einem der Ansprüche 1 bis 5 mit einer erhöhten Selektivität gegenüber Faktor Xa, **dadurch gekennzeichnet, dass** man außerdem die Disaccharid- und Tetrasaccharidfraktionen mittels Chromatographie, insbesondere an Säulen, die mit einem Gel des Polyacrylamid-Agarose-Typs gefüllt sind, entfernt.

21. Herstellungsverfahren nach einem der Ansprüche 8 bis 20 für Oligosaccharidgemische nach einem der Ansprüche 1 bis 7, **dadurch gekennzeichnet, dass** man als Ausgangsprodukt ein niedermolekulares Heparin oder ein sehr niedermolekulares Heparin (1500 bis 3000 Da) mit einer Anti-Xa-Aktivität zwischen 100 und 140 IU/mg anstelle des sehr niedermolekularen Heparins nach Anspruch 8 verwendet.

22. Herstellungsverfahren nach einem der Ansprüche 8 bis 20 für Oligosaccharidgemische nach einem der Ansprüche 1 bis 7, **dadurch gekennzeichnet, dass** man als Ausgangsprodukt ein sehr niedermolekulares Heparin (1500 bis 4000 Da) mit einer Anti-Xa-Aktivität zwischen 100 und 140 IU/mg anstelle des sehr niedermolekularen Heparins nach Anspruch 8 verwendet.

23. Herstellungsverfahren nach Anspruch 21, **dadurch gekennzeichnet, dass** das niedermolekulare Heparin aus Enoxaparin, Fraxiparin, Fragmin, Innohep (oder Logiparin), Normiflo, Embollex (oder Sandoparin), Fluxum (oder Minidalton), Clivarin und Hibor ausgewählt wird.

24. Arzneimittel, **dadurch gekennzeichnet, dass** es ein Oligosaccharidgemisch nach einem der Ansprüche 1 bis 7 umfasst.

25. Arzneimittel nach Anspruch 24 zur Behandlung oder Vorbeugung von venösen und arteriellen Thrombosen, tiefer Venenthrombose, Lungenembolie, instabiler Angina pectoris, Myokardinfarkt, Herzischämie, Verschlusskrankheiten der peripheren Arterien und Vorhofflimmern, der Proliferation glatter Muskelzellen, von Atherosklerose und Arteriosklerose, Krebs durch Veränderung der Angiogenese und Wachstumsfaktoren sowie diabetischen Störungen, wie diabetischen Retinopathien und Nephropathien.

26. Pharmazeutische Zusammensetzung, die mindestens ein Arzneimittel nach Anspruch 24 und einen oder mehrere pharmazeutisch inerte Excipienten, Vehikel oder Additive enthält.

27. Pharmazeutische Zusammensetzung nach Anspruch 26, **dadurch gekennzeichnet, dass** sie aus einer Lösung besteht, die auf subkutanem oder intravenösem Weg injiziert werden kann.

28. Pharmazeutische Zusammensetzung nach Anspruch 26, **dadurch gekennzeichnet, dass** sie aus einer Formulierung zur Inhalation auf dem Lungenweg besteht.

29. Pharmazeutische Zusammensetzung nach Anspruch 26, **dadurch gekennzeichnet, dass** sie aus einer Formulierung für den oralen Weg besteht.
